(19)

Europäisches
Patentamt
European
Patent Office
Office européen
des brevets

(11) **EP 4 226 943 A1**

(12) **EUROPEAN PATENT APPLICATION**
published in accordance with Art. 153(4) EPC

(43) Date of publication:
**16.08.2023 Bulletin 2023/33**

(21) Application number: **21877773.8**

(22) Date of filing: **08.10.2021**

(51) International Patent Classification (IPC):
**A61K 39/395** *(2006.01)*   **A61P 29/00** *(2006.01)*
**A61P 37/02** *(2006.01)*   **C07K 16/28** *(2006.01)*
**C12N 15/13** *(2006.01)*

(52) Cooperative Patent Classification (CPC):
**A61K 39/395; A61P 29/00; A61P 37/02;**
**C07K 16/00; C07K 16/28**

(86) International application number:
**PCT/JP2021/037471**

(87) International publication number:
**WO 2022/075474 (14.04.2022 Gazette 2022/15)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **09.10.2020 JP 2020171278**

(71) Applicants:
• **Keio University**
  **Tokyo, 108-8345 (JP)**
• **Takeda Pharmaceutical Company Limited**
  **Osaka-shi, Osaka 541-0045 (JP)**

(72) Inventors:
• **TAKEUCHI, Tsutomu**
  **Tokyo 160-8582 (JP)**
• **YOSHIMURA, Akihiko**
  **Tokyo 160-8582 (JP)**
• **SUZUKI, Katsuya**
  **Tokyo 160-8582 (JP)**

• **TAKESHITA, Masaru**
  **Tokyo 160-8582 (JP)**
• **KOJIMA, Marenori**
  **Tokyo 160-8582 (JP)**
• **KASSAI, Yoshiaki**
  **Fujisawa-shi, Kanagawa 251-0012 (JP)**
• **KORO, Taku**
  **Fujisawa-shi, Kanagawa 251-0012 (JP)**
• **SEKIYA, Keiko**
  **Fujisawa-shi, Kanagawa 251-0012 (JP)**
• **YOSHIHARA, Tomoki**
  **Fujisawa-shi, Kanagawa 251-0012 (JP)**
• **ADACHI, Ryutaro**
  **Fujisawa-shi, Kanagawa 251-0012 (JP)**

(74) Representative: **Hoffmann Eitle**
**Patent- und Rechtsanwälte PartmbB**
**Arabellastraße 30**
**81925 München (DE)**

Remarks:
The complete document including Reference
Table(s) and the Sequence Listing(s) can be
downloaded from the EPO website

(54) **THERAPEUTIC AGENT FOR IMMUNE/INFLAMMATORY DISEASE**

(57)   The present invention provides a therapeutic
agent for an immune/inflammatory disease comprising
an anti-human TIGIT antibody, which activates a suppressive immune checkpoint molecule (TIGIT), as an ac-
tive ingredient. Also, provided is an antibody having the
following characteristics a) and b):
a) the third CDR of heavy chain variable region comprises
the amino acid sequence of SEQ ID NO: 4
b) the third CDR of light chain variable region comprises
the amino acid sequence of SEQ ID NO: 6.

[Fig. 4]

**EP 4 226 943 A1**

**Description**

[Technical Field]

The present invention relates to a therapeutic agent for an immune/inflammatory disease comprising an antibody against human TIGIT. The present invention also relates to an anti-human TIGIT antibody having a specific primary structure, that can have a therapeutic activity for an immune/inflammatory disease, and the like.

(Background of the Invention)

In recent years, a medicament targeting an immune checkpoint protein, particularly an anticancer drug has been actively developed. Immune checkpoint proteins are factors that prevent development of an immune/inflammatory disease caused by abnormal activation of an immune system, and the like. PD-1 (programmed cell death protein 1, also known as CD279), PD-L1 (programmed cell death ligand 1, also known as B7-H1/CD274), PD-L2 (programmed cell death ligand 2, also known as B7-DC/CD273) and CTLA-4 (cytotoxic T-lymphocyte-associated antigen 4, also known as CD152) and the like are known as major molecules of the immune checkpoint proteins. In the meantime, TIGIT (T-cell immunoreceptor with immunoglobulin and ITIM domains, also known as zB7R1) can be mentioned as an immune checkpoint protein other than the above-mentioned molecules. TIGIT is an immune checkpoint receptor present in a natural-killer (NK) cell, cytotoxic T cell (CTL), memory-T cell, regulatory T cell (Treg cell), T follicular helper cell (Tfh cell) and the like. It is known that in CTL and NK cell, immune activation is suppressed by an interaction between TIGIT and any of two ligands thereof, CD155(PVR) or CD112 (Nectin2), whereas TIGIT expression in Treg enhances a potential for suppressing an immune response by their interaction. The immunosuppressive action due to the binding of TIGIT and CD155 or CD112 occurs by competitive inhibition against that of CD226, which is an immune activating receptor that is expressed in a cytotoxic T cell and NK cell, and the same ligands (namely, CD155 ligand and CD112 ligand).

A method for treating an immune/inflammatory disease using an agonist for TIGIT or an agonistic anti-TIGIT antibody via suppression of the function of TIGIT-positive T cells, focusing on signal transduction through TIGIT, has been reported (Patent Literatures 1, 2). However, no medicament containing an agonistic anti-TIGIT antibody has been approved, and the development of an anti-TIGIT antibody excellent in therapeutic effects, safety, and the like has been desired.

[Citation List]

[Patent Literature]

[PTL 1]
WO 2006/124667
[PTL 2]
WO 2009/126688

[Summary of Invention]

[Problem to Be Solved by the Invention]

Therefore, it is a problem to be solved by the present invention to provide an agonistic anti-human TIGIT antibody capable of binding to human TIGIT and enhancing the signal through TIGIT, and to provide a novel therapeutic agent for an immune/inflammatory disease containing an agonistic antibody against human TIGIT.

[Means of Solving the Problem]

In the course of studies relating to the search of targets for drug discovery based on elucidation of a pathogenic mechanism of an immune disease, the present inventors found out that TIGIT expression levels elevated in patients with rheumatoid arthritis. It was confirmed that TIGIT expression levels in the disease were significantly high, particularly in Tfh cell, peripheral helper T cell (peripheral T; Tph cell), and Treg cell, which cells show high TIGIT expression levels even in a healthy individual. Furthermore, the present inventors found out that TIGIT expression level in Tph cells of patients with rheumatoid arthritis also correlates with activity of the disease. The present inventors have made intensive studies to solve the above-mentioned problem, based on the idea that a therapeutic drug for immune/inflammatory diseases such as rheumatoid arthritis may be developed by using TIGIT as a target. At first, the present inventors established a plurality of mouse anti-human TIGIT (anti-hTIGIT) antibodies, and selected plural antibodies having a particularly high agonistic activity from among the established antibodies. Furthermore, the present inventors verified suppression of proliferation of Tfh cells, which show high TIGIT expression level, by the selected agonistic antibodies, and found that a specific antibody (Clone M1-8) could suppress proliferation of Tfh cells, whereby the present invention has been completed.

That is, the present invention provides the following.

[1] A therapeutic agent for an immune/inflammatory disease comprising an anti-human TIGIT antibody, which activates a suppressive immune checkpoint molecule (TIGIT), as an active ingredient.

[1'] A method for treating an immune/inflammatory disease in a mammal, comprising administering to the mammal an effective amount of an anti-human TIGIT antibody that activates a suppressive immune checkpoint molecule (TIGIT).

[1"] An anti-human TIGIT antibody that activates a suppressive immune checkpoint molecule (TIGIT) for use in the treatment of an immune/inflammatory disease.

[2] The therapeutic agent according to [1], which has Tfh cell suppressing action.
An agent for suppressing Tfh cell comprising an anti-human TIGIT antibody that activates a suppressive immune checkpoint molecule (TIGIT).

[3] The therapeutic agent according to [1] or [2], which has Treg cell activating action.
An agent for activating Treg cell comprising an anti-human TIGIT antibody that activates a suppressive immune checkpoint molecule (TIGIT).

[4] The therapeutic agent according to any of [1] to [3], wherein the immune/inflammatory disease is selected from the group consisting of rheumatoid arthritis, systemic lupus erythematosus, Sjogren's syndrome, systemic scleroderma, polymyositis, dermatomyositis, IgG4-associated disease, Takayasu arteritis, giant cell arteritis, polyarteritis nodosa, ANCA-associated vasculitis, mixed connective tissue disease, spondylarthritis, Behcet's disease, adult Still's disease, multiple sclerosis, optic nerve myelitis, myasthenia gravis, primary biliary cirrhosis, non-alcoholic fatty liver diseases, primary sclerosing cholangitis, autoimmune hepatitis, ulcerative colitis, Crohn's disease, psoriasis (psoriatic arthritis), vitiligo vulgaris, bullous pemphigoid, circular shape alopecia, idiopathic dilated cardiomyopathy, type 1 diabetes, Graves' disease, chronic thyroiditis, IgA nephropathy, membranous nephropathy, hemolytic anemia, and idiopathic thrombocytopenic purpura.

[5] The therapeutic agent according to any of [1] to [4], wherein the anti-human TIGIT antibody is an antibody binding to human TIGIT (SEQ ID NO: 1).

[6] The therapeutic agent according to [5], wherein in the antibody,

a) the third CDR of the heavy chain variable region comprises the amino acid sequence of SEQ ID NO: 4, and
b) the third CDR of the light chain variable region comprises the amino acid sequence of SEQ ID NO: 6.

[7] The therapeutic agent according to [5] or [6], wherein the antibody comprises

a) a heavy chain variable region comprising the following:

i) the first CDR comprising the amino acid sequence of SEQ ID NO: 2;
ii) the second CDR comprising the amino acid sequence of SEQ ID NO: 3; and

b) a light chain variable region comprising the following:

i) the first CDR comprising the amino acid sequence of SEQ ID NO: 5;
ii) the second CDR comprising the amino acid sequence of tyrosine-alanine-serine.

[8] The therapeutic agent according to any of [5] to [7], wherein the antibody further comprises Fc domain.
[9] The therapeutic agent according to [8], wherein the Fc domain is derived from human.
[10] The therapeutic agent according to [8] or [9], wherein the Fc domain is a mutated Fc domain.
[11] An antibody:

a) whose third CDR of the heavy chain variable region comprises the amino acid sequence of SEQ ID NO: 4, and
b) whose third CDR of the light chain variable region comprises the amino acid sequence of SEQ ID NO: 6.

[12] The antibody according to [11], which binds to human TIGIT (SEQ ID NO: 1).

[13] The antibody according to [11] or [12], comprising

a) a heavy chain variable region comprising the following:

i) the first CDR comprising the amino acid sequence of SEQ ID NO: 2;
ii) the second CDR comprising the amino acid sequence of SEQ ID NO: 3; and

b) a light chain variable region comprising the following:

i) the first CDR comprising the amino acid sequence of SEQ ID NO: 5;
ii) the second CDR comprising the amino acid sequence of tyrosine-alanine-serine.

[14] The antibody according to any of [11] to [13], which further comprises Fc domain.

[15] The antibody according to [14], wherein the Fc domain is derived from human.

[16] The antibody according to [14] or [15], wherein the Fc domain is a mutated Fc domain.

[17] An isolated nucleic acid encoding the antibody according to any of [11] to [16].

[18] A host cell comprising the isolated nucleic acid according to [17].

[19] A method of producing the antibody according to any of [11] to [16], comprising a step for culturing the host cell according to [18] under conditions that allows the production of the antibody.

[Effects of the Invention]

The present invention provides an antibody that binds to human TIGIT. Since the antibody can be a therapeutic drug for an immune/inflammatory disease, the present invention is useful for the treatment of an immune/inflammatory disease by using the therapeutic drug.

[Brief Description of Drawings]

[Fig. 1]

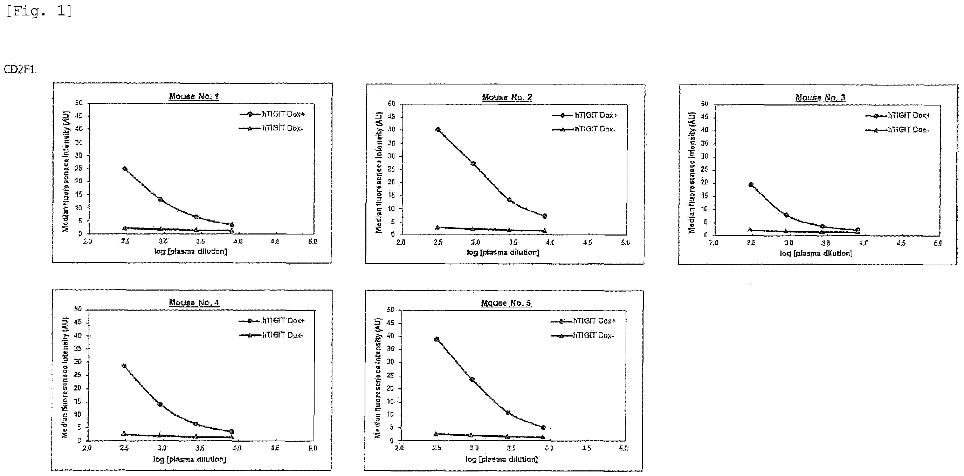

Fig. 1 shows results of the measurement of antibody titer in the antisera of mice immunized with a recombinant human TIGIT-His-mFc protein. The vertical axis of each graph shows the median value of fluorescence intensity (Median fluorescence intensity). The horizontal axis of each graph shows log [plasma dilution].
[Fig. 2]

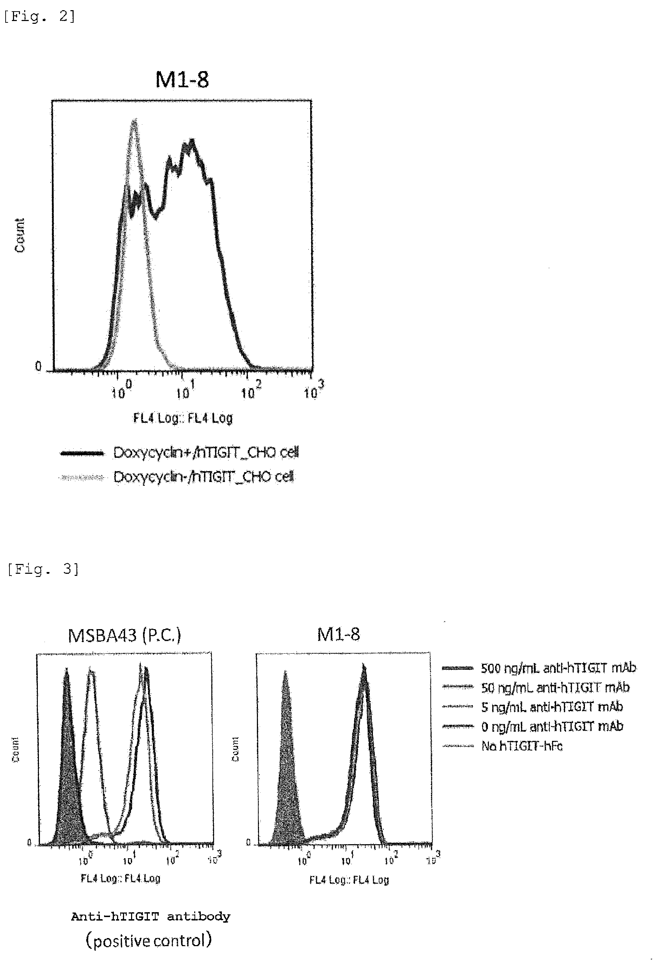

Fig. 2 shows binding activity of M1-8 antibody in the culture supernatant against TIGIT-expressing cells which expression being induced by doxycycline.
[Fig. 3]
Fig. 3 shows inhibitory activity of M1-8 antibody in the culture supernatant on binding between a recombinant TIGIT and PVR-expressing cells.
[Fig. 4]

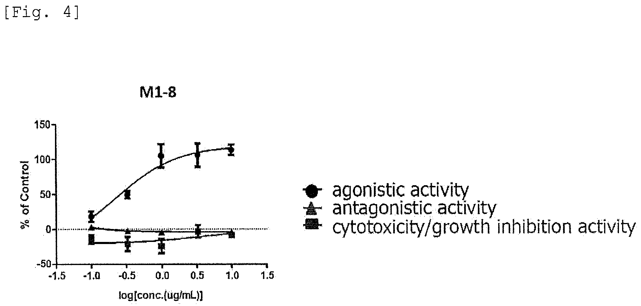

Fig. 4 shows biological activities (agonistic activity, antagonistic activity, cytotoxic activity) of purified M1-8 antibody.
[Fig. 5]

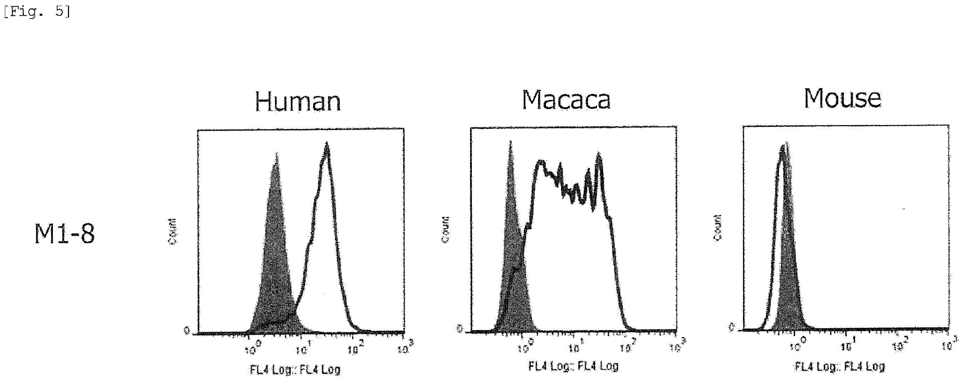

Fig. 5 shows binding activity of purified M1-8 antibody against human, *Macaca fascicularis,* and mouse TIGIT-expressing cells.
[Fig. 6]

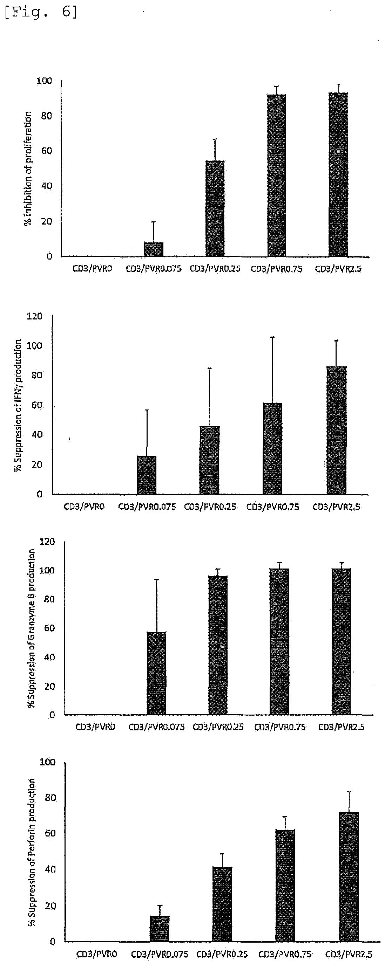

Fig. 6 shows, from the top, proliferation-suppressing effect of (% inhibition of proliferation) and cytokines (IFN$\gamma$, Granzyme B, Perforin) production-suppressing effect (% suppression of cytokines production) by TIGIT stimulation against CD8-positive cells. The horizontal axis of each graph shows, from the left, CD3/PVR0, CD3/PVR0.075, CD3/PVR0.025, CD3/PVR0.75, and CD3/PVR2.5.
[Fig. 7-1]

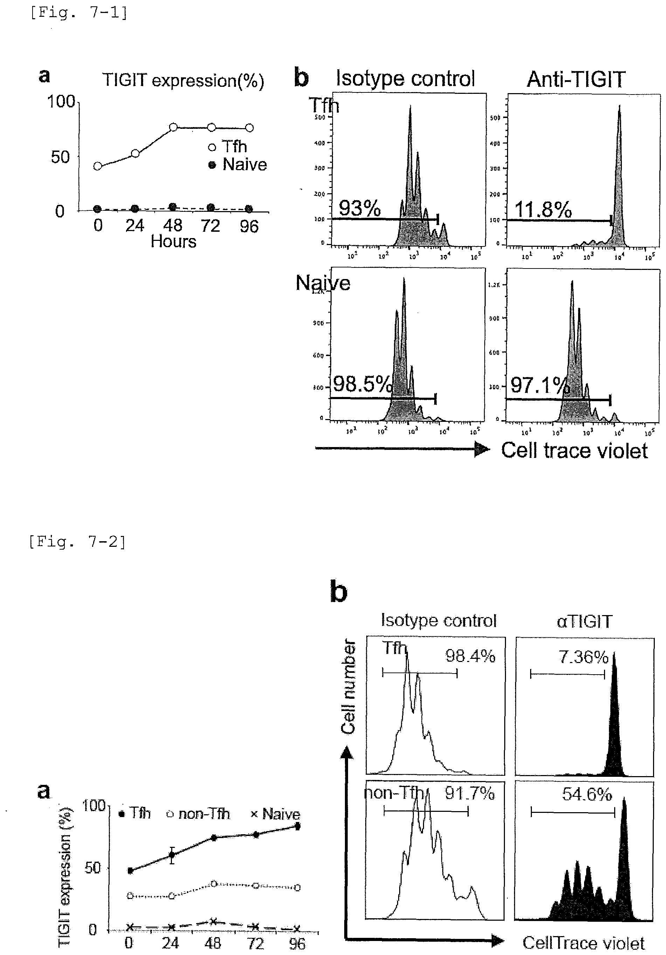

Fig. 7-1a shows how TIGIT expression changes by stimulation in cells showing intrinsically low TIGIT expression (CD4+ naive T cells) and cells showing intrinsically high expression (Tfh cells). Fig 7-1b shows that stimulation by an anti-TIGIT agonist antibody suppresses proliferation of Tfh cells.
[Fig. 7-2]
Fig. 7-2a shows time course changes of TIGIT expression in Tfh cells, non-Tfh cells and CD4+ naive T cells by activating stimulation. Non-Tfh cells showed TIGIT expression intermediate between Tfh cells and naive T cells. Fig. 7-2b shows that stimulation by an anti-TIGIT agonistic antibody suppresses proliferation of Tfh cells and non-Tfh cells, but such effect is more remarkable to Tfh cells.

[Fig. 8]

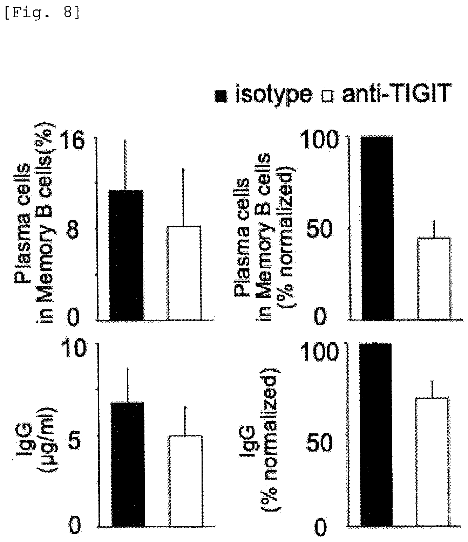

Fig. 8 shows that stimulation against Tfh cells by an anti-TIGIT agonistic antibody suppresses B cell activation via Tfh cells. The stimulation against B cells from Tfh cells is evaluated by proportion of plasma cells and IgG concentration in the culture supernatant. Absolute values and relative values when the results of isotype are defined as 100 are shown.

[Fig. 9-1]

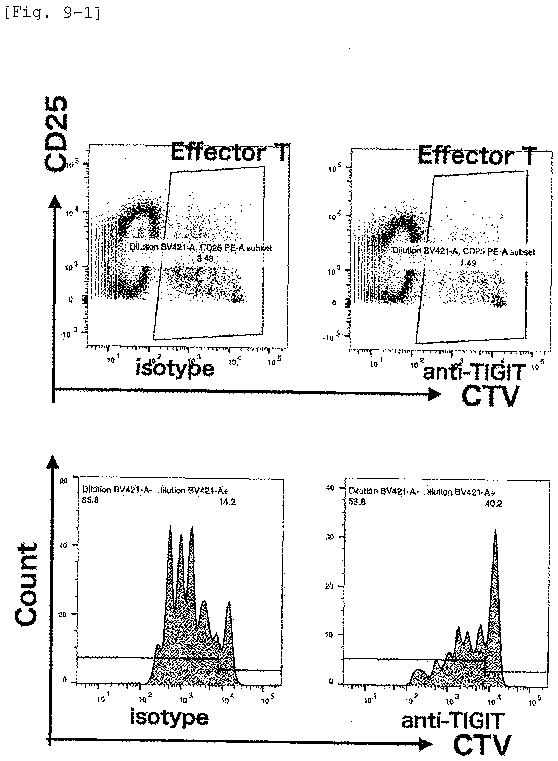

Fig. 9-1 shows that stimulation against Treg cells by an anti-TIGIT agonistic antibody suppressed cell proliferation of effector T cells.

[Fig. 9-2]

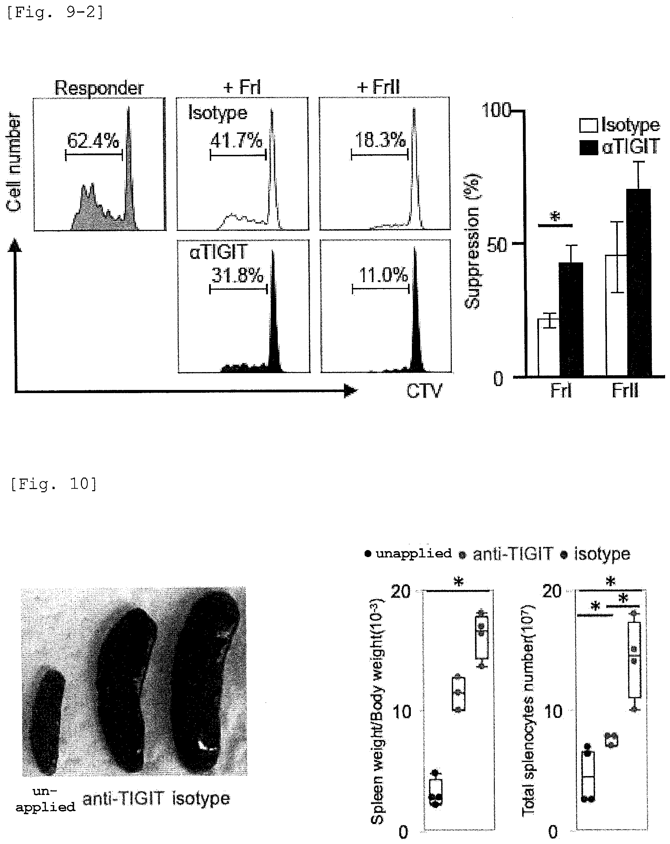

Fig. 9-2 shows that stimulation against Treg cells (FrI and FrII) by an anti-TIGIT agonistic antibody suppresses proliferation of non-Treg responder CD4+ T cells (CD25-), and that the suppressing effect is more remarkable in Treg cells (FrI). The vertical axis of the right bar graph shows suppression rate $(\%)=[1-(\text{proliferation of co-cultured responder T cells})/(\text{proliferation of solely cultured responder T cells})]\times 100$. *:$p<0.05$

[Fig. 10]

Fig. 10 shows that an anti-TIGIT agonistic antibody improves splenomegaly in imiquimod-induced lupus model mice and suppresses proliferation of spleen lymphocytes. The results were compared between three groups, unapplied, anti-TIGIT and isotype (n=4, 3 and 4, respectively).

[Fig. 11-1]

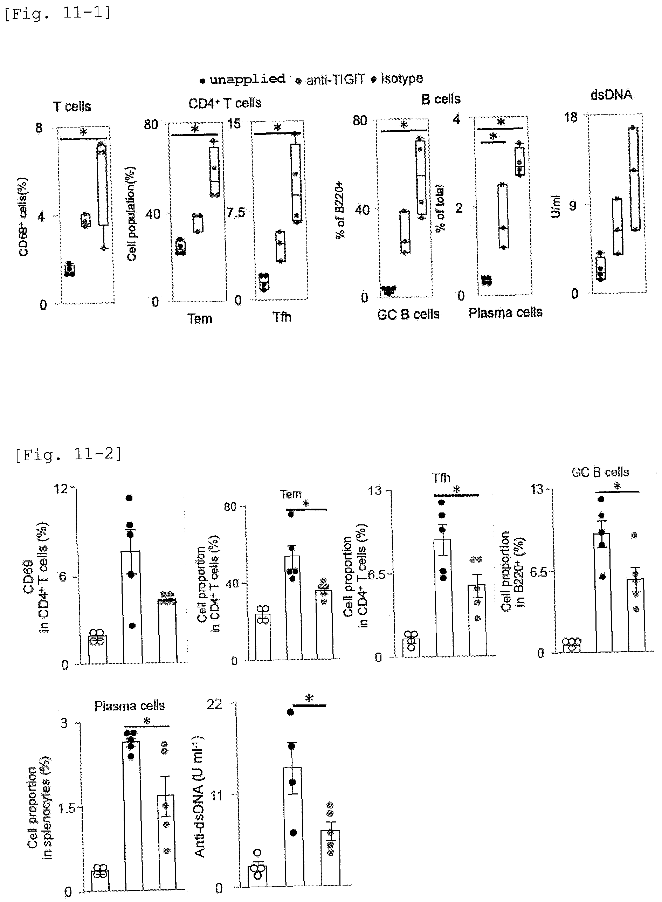

Fig. 11-1 shows comparison between the same three groups as shown in Fig. 10 of, from the left, proportion of CD69+ (activated) T cells in T cells, proportions of CD4+ Effector memory T cells and Tfh cells in CD4+ T cells, proportions of germinal center B cells and plasma cells in B cells, in the spleen of imiquimod-induced lupus model mice, as well as anti-dsDNA antibody content in the plasma of the mice. Since blood could not be obtained from a mouse of the isotype group, the group is n=3 only for anti-dsDNA antibody. The box and whisker plots of each data show unapplied group, anti-TIGIT-applied group and isotype-applied group, respectively, from the left. *:$p<0.05$

[Fig. 11-2]

Fig. 11-2 shows effects of the administration of an anti-TIGIT agonistic antibody on immune response in imiquimod-induced lupus model mice. The upper panel shows, from the left, proportion of CD69+ (activated) T cells in CD4+ T cells, proportions of CD4+ Effector memory T (Tem) cells and Tfh cells in CD4+ T cells, and proportion of germinal center (GC) B cells in B220$^+$ cells, in the spleen. The lower panel shows, from the left, proportion of plasma cells in splenocytes, and anti-dsDNA antibody content in plasma of the mice. The results were compared between three groups (administered with isotype alone, imiquimod-applied + administered with anti-TIGIT, and imiquimod-applied + administered with isotype, n=4, 5, and 5, respectively). In each bar graph, the bars show the unapplied group, the isotype-applied group and the anti-TIGIT-applied group, respectively, from the left. *:$p<0.05$

[Fig. 12]

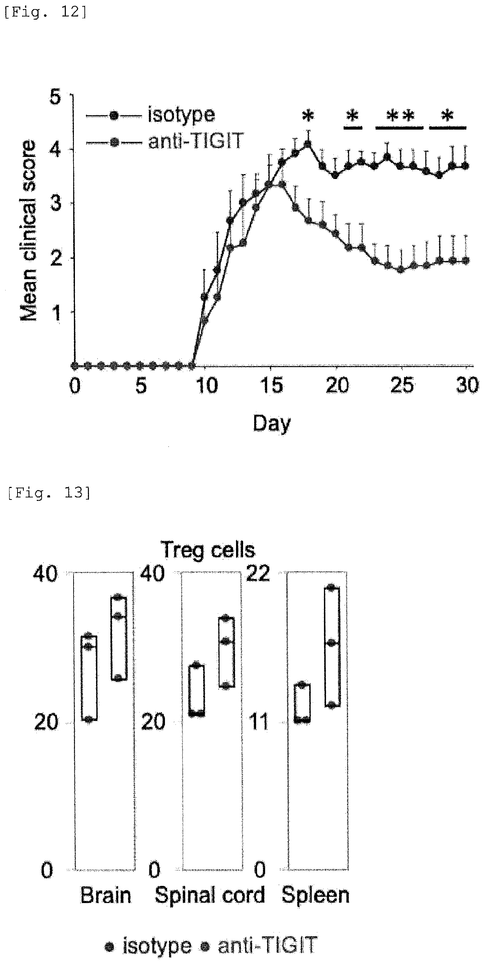

Fig. 12 shows that an anti-TIGIT agonistic antibody improves clinical score of EAE mice. Six mice per group were tested, and clinical scores are shown in chronological order for group administered with isotype and group administered with anti-TIGIT agonistic antibody, respectively.

[Fig. 13]

Fig. 13 shows that Treg cells in the brain, spinal cord and spleen of EAE mice are proliferated by administration of an anti-TIGIT agonistic antibody. Three mice per group were tested, cells in the brain, spinal cord and spleen were stained for group administered with isotype and group administered with anti-TIGIT agonistic antibody, respectively, and the proportions of Treg cells were compared. The box and whisker plots of each data show anti-TIGIT-applied group and isotype-applied group, respectively, from the left.

(Detailed Description of the Invention)

Antibody binding to human TIGIT or fragment thereof

The present invention provides an antibody, which has complementarity determination regions (CDRs) comprising specific sequences and binds to human TIGIT, or a fragment thereof. As used hereinafter, the term "the antibody of the present invention" encompasses the above-mentioned antibody and a fragment thereof. The antibody of the present invention may be isolated. As used herein, "isolated" means conditions that a specific component (e.g., the antibody of the present invention, a nucleic acid encoding the antibody, etc.) is identified, separated or recovered from a component of its natural environment (e.g., cell, etc.).

The antibody of the present invention comprises the third CDR (also referred to as CDR3) of heavy chain variable region comprising the amino acid sequence represented by SEQ ID NO: 4, and the third CDR of light chain variable region comprising the amino acid sequence represented by SEQ ID NO: 6. Also, in one embodiment, the antibody of the present invention comprises i) the first CDR (also referred to as CDR1) of heavy chain variable region comprising the amino acid sequence represented by SEQ ID NO: 2 and ii) the second CDR (also referred to as CDR2) of heavy chain variable region comprising the amino acid sequence represented by SEQ ID NO: 3, as well as iii) the first CDR of light chain variable region comprising the amino acid sequence represented by SEQ ID NO: 5 and iv) the second CDR of light chain variable region comprising the amino acid sequence represented by tyrosine-alanine-serine (YAS). The heavy chain variable region comprising the CDR1-CDR3 of heavy chain variable region represented by SEQ ID NOs: 2-4, respectively, includes, as one embodiment, but is not limited to, that comprising the amino acid sequence represented by SEQ ID NO: 7. Also, the light chain variable region comprising the CDR1-CDR3 of light chain variable region represented by SEQ ID NO: 5, YAS and SEQ ID NO: 6, respectively, includes, as one embodiment, but is not limited to, that comprising the amino acid sequence represented by SEQ ID NO: 8.

Therapeutic agent for immune/inflammatory disease comprising anti-human TIGIT agonistic antibody

The present invention provides a therapeutic agent for an immune/inflammatory disease comprising an anti-human TIGIT antibody, which activates a suppressive immune checkpoint molecule, human TIGIT, as an active ingredient (hereinafter sometimes referred to as "the therapeutic agent of the present invention"). An antibody that has complementarity determination regions (CDRs) comprising specific sequences and binds to human TIGIT or a fragment thereof can be mentioned as one embodiment of the antibody, which is an active ingredient of the therapeutic agent of the present invention. As used hereinafter, the term "anti-human TIGIT agonistic antibody" encompasses the above-mentioned antibody, which is an active ingredient of the present invention, and a fragment thereof.

As shown in the following Examples, since an anti-human TIGIT agonistic antibody has a suppressive action against T follicular helper (Tfh) cells (hereinafter sometimes referred to as "Tfh cell-suppressing action"), it has a therapeutic effect on an immune/inflammatory disease caused by activation of Tfh cells. In addition, the anti-human TIGIT agonistic antibody has an activating action against Treg cells (hereinafter sometimes referred to as "Treg cell-activating action"). Therefore, in another embodiment of the present invention, provided is an agent for suppressing Tfh cell comprising an anti-human TIGIT agonistic antibody, or a method of suppressing Tfh cell comprising a step for contacting an anti-human TIGIT agonistic antibody with Tfh cell. Further, in another embodiment, provided is an agent for activating Treg cell comprising an anti-human TIGIT agonistic antibody, or a method of activating Treg cell comprising contacting an anti-human TIGIT agonistic antibody with Treg cell.

As used herein, the "immune/inflammatory disease" means a disease accompanied with inflammation due to breakdown of immune tolerance. The immune/inflammatory diseases as target diseases of the therapeutic agent of the present invention include, for example, rheumatoid arthritis, systemic lupus erythematosus, Sjogren's syndrome, systemic scleroderma, polymyositis, dermatomyositis, IgG4-associated disease, Takayasu arteritis, giant cell arteritis, polyarteritis nodosa, ANCA-associated vasculitis, mixed connective tissue disease, spondylarthritis, Behcet's disease, adult Still's disease, multiple sclerosis, optic nerve myelitis, myasthenia gravis, primary biliary cirrhosis, non-alcoholic fatty liver diseases, primary sclerosing cholangitis, autoimmune hepatitis, ulcerative colitis, Crohn's disease, psoriasis (psoriatic arthritis), vitiligo vulgaris, bullous pemphigoid, circular shape alopecia, idiopathic dilated cardiomyopathy, type 1 diabetes, Graves' disease, chronic thyroiditis, IgA nephropathy, membranous nephropathy, hemolytic anemia, and idiopathic thrombocytopenic purpura. The subjects of administration of the therapeutic agent of the present invention include, for example, primate animals. The primate animals include lemur, loris, tupai, monkey (e.g., *Macaca fascicularis* etc.), and human, preferably human. Therefore, a therapeutic method comprising administering to the primate animals an effective amount of an anti-human TIGIT agonistic antibody or the therapeutic agent of the present invention is also encompassed within the present invention.

As used herein, the "Tfh cell-suppressing action" encompasses both Tfh cell proliferation-suppressing action and Tfh cell function-suppressing action. The Tfh cell functions include, for example, maturation and activation of B cells, antibody production-promoting effect and the like. The "Treg cell-activating action" encompasses both Treg cell proliferation-promoting action and Treg cell function-activating action. The Treg cell functions include, for example, suppression of autoimmune response via suppression of effector T cells, suppressive function against excess inflammation via secretion of anti-inflammatory cytokines such as IL-10 and TGFβ and the like, and the like.

(Anti-human TIGIT agonistic antibody)

In one embodiment, the anti-human TIGIT agonistic antibody comprises the third CDR (also referred to as CDR3) of heavy chain variable region comprising the amino acid sequence represented by SEQ ID NO: 4, and the third CDR of light chain variable region comprising the amino acid sequence represented by SEQ ID NO: 6. Also, the anti-human TIGIT agonistic antibody further may comprise i) the first CDR (also referred to as CDR1) of heavy chain variable region comprising the amino acid sequence represented by SEQ ID NO: 2 and ii) the second CDR (also referred to as CDR2) of heavy chain variable region comprising the amino acid sequence represented by SEQ ID NO: 3, as well as iii) the first CDR of light chain variable region comprising the amino acid sequence represented by SEQ ID NO: 5 and iv) the second CDR of light chain variable region comprising the amino acid sequence represented by tyrosine-alanine-serine (YAS). The heavy chain variable region comprising the CDR1-CDR3 of heavy chain variable region represented by SEQ ID NOs: 2-4, respectively, includes, as one embodiment, but is not limited to, that comprising the amino acid sequence represented by SEQ ID NO: 7. Also, the light chain variable region comprising the CDR1-CDR3 of light chain variable region represented by SEQ ID NO: 5, YAS and SEQ ID NO: 6, respectively, includes, as one embodiment, but is not limited to, that comprising the amino acid sequence represented by SEQ ID NO: 8.

The anti-human TIGIT agonistic antibody can activate TIGIT by binding thereto (in other words, exert agonistic activity against TIGIT). As used herein, "activating TIGIT" means that signaling via TIGIT is activated by binding the antibody thereto, as compared to a control without binding the antibody (including a group contacted with an antibody without TIGIT-activating action), or before binding the antibody. Also, as used herein, the "anti-human TIGIT agonistic antibody" means an antibody that activates signaling via TIGIT by binding thereto, as compared to a control without binding the antibody or before binding the antibody. Such activation of the signaling via TIGIT can be evaluated by any of methods known per se, such as luciferase assay and the like. In addition, an anti-human TIGIT agonistic antibody other than the above-mentioned antibody comprising the specific CDR1-CDR3 can be also screened by such evaluation method.

As used herein, the term "binding" means "having an ability to bind", i.e., an ability to form a complex with noncovalent bond(s) with one or more other molecule(s). Examples of the complex of the present invention include a complex of the anti-human TIGIT agonistic antibody and TIGIT. The anti-human TIGIT agonistic antibody binds to a human TIGIT protein consisting of the amino acid sequence represented by SEQ ID NO: 1 (NCBI Reference Sequence: NP_776160.2). In one embodiment, the anti-human TIGIT agonistic antibody binds to not only a human TIGIT protein but also a TIGIT of a primate other than human (e.g., *Macaca fascicularis* etc.), but does not bind to a TIGIT of a rodent (e.g., mouse etc.). Therefore, hereinafter, the "human TIGIT" may be also referred to as "primate TIGIT". Such binding ability of the antibody can be evaluated by any of the methods known per se. For example, such evaluation can be performed by contacting TIGIT and the antibody, and defecting or measuring the antibody that binds to TIGIT.

The anti-human TIGIT agonistic antibody may be any antibody or a fragment thereof, as long as it comprises the above-mentioned CDR3 of heavy chain variable region and the CDR3 of light chain variable region. The class of the antibody is not particularly limited, and may be any isotype such as IgG, IgM, IgA, IgD, IgE and the like. Preferably, the class is IgG or IgM, more preferably IgG, in view of easiness of purification and the like. The fragment of the antibody is not particularly limited, as long as it comprises the above-mentioned CDR3 of heavy chain variable region and the CDR3 of light chain variable region and binds to human TIGIT, for example, antibody fragments having the above-mentioned CDRs such as Fab, Fab', F(ab')$_2$ and the like can be mentioned.

Also, it is preferable that the anti-human TIGIT agonistic antibody has an Fc domain such as a full-body antibody and an antibody in which Fab region and Fc domain are conjugated. The Fc domain may be wild-type or variant. As is known in the art, an antibody Fc domain interacts with many Fc receptors and ligands, and confers an important function called as effector function. Such Fc receptors include, but are not limited to, the following: FcγRI(CD64) including isoforms FcγRIa, FcγRIb and FcγRIc; FcγRII(CD32) including isoforms FcγRIIa (including allotypes H131 and R131), FcγRIIb (including FcγRIIb-1 and FcγRIIb-2) and FcγRIIc; and FcγRIII(CD16) including isoforms FcγRIIIa (including allotypes V158 and F158, and associated with antibody-dependent cellular cytotoxicity (ADCC)) and FcγRIIIb (including allotypes FcγRIIIb-NA1 and FcγRIIIb-NA2); FcRn (neonatal receptor), C1q (complement protein involved in complement-dependent cytotoxicity (CDC)), as well as FcRn (neonatal receptor involved in serum half-life) (in human). Suitable amino acid mutation can be made at one or more positions, as outlined in the following: for example, U.S. Patent Application 11/841,654 and the references cited therein, US 2004/013210, US 2005/0054832, US 2006/0024298, US 2006/0121032, US 2006/0235208, US 2007/0148170.

The moieties other than the above-mentioned CDRs of the anti-human TIGIT agonistic antibody (e.g., variable region moieties other than CDRs, constant region, Fc domain and the like) may consist of any amino acid sequences, as long as the antibody has an agonistic activity against TIGIT, and may be derived from any animals. Examples of the animals include mammals such as mouse, rat, hamster, guinea pig, dog, monkey, orangutan, chimpanzee, human and the like. Preferably, the moieties are derived from human.

The anti-human TIGIT agonistic antibody can be produced in the manner of genetic engineering using a nucleic acid or vector encoding the antibody or a fragment thereof. For example, a nucleic acid encoding the anti-human TIGIT agonistic antibody can be introduced into a host cell to express the antibody or a fragment thereof, and the antibody or a fragment thereof can be isolated by a method known per se. Examples of the isolation methods include affinity column using protein A and the like, other chromatographic columns, filter, ultrafiltration, salting out, dialysis and the like. These methods may be appropriately combined. The antibody fragment can be also produced by treating the full-body antibody produced as described above with an enzyme such as papain, pepsin and the like.

(Nucleic acid encoding the anti-human TIGIT agonistic antibody)

The present invention provides an isolated nucleic acid encoding the antibody of the present invention (hereinafter sometimes referred to as "the nucleic acid of the present invention"). The nucleic acid of the present invention encompasses a nucleic acid encoding the heavy chain of the antibody of the present invention and a nucleic acid encoding the light chain of the antibody, a nucleic acid encoding a fragment of the antibody and the like. The nucleic acid encoding the antibody of the present invention, the anti-human TIGIT agonistic antibody or a fragment thereof may be DNA or RNA, or DNA/RNA chimera, preferably DNA. Also, the nucleic acid may be double-stranded or single stranded. When the nucleic acid is double-stranded, it may be double-stranded DNA, double-stranded RNA or DNA:RNA hybrid. The nucleic acid encoding the antibody or a fragment thereof may contain naturally-occurring nucleotide, modified nucleotide, nucleotide analog, or a mixture thereof, as long as the nucleic acid can be expressed as a polypeptide *in vitro* or in a cell.

A nucleic acid encoding an antibody or a fragment thereof (e.g., the nucleic acid of the present invention) can be constructed by a method known per se. For example, a DNA encoding full-length or a part of the anti-human TIGIT agonistic antibody can be constructed by designing a base sequence encoding an amino acid sequence of an anti-human TIGIT agonistic antibody, based on the amino acid sequence described in Sequence Listing, and chemically synthesizing the DNA strand, or ligating partially overlapping oligo DNA short strands synthesized utilizing PCR method or Gibson Assembly method.

Also, the nucleic acid encoding the antibody or a fragment thereof can be integrated into an expression vector. Therefore, provided is an expression vector containing any of the above-mentioned nucleic acids encoding the antibody or a fragment thereof. In this case, the nucleic acid encoding the antibody heavy chain and the nucleic acid encoding the antibody light chain may be integrated into separate expression vectors or integrated into a single expression vector. When these two kinds of nucleic acids are integrated into a single expression vector, they may be integrated via a sequence that allows polycistronic expression. The plural genes integrated in a kind of expression vector can be more efficiently expressed by use of the sequence that allows polycistronic expression. Examples of the sequence that allows polycistronic expression include 2A sequence (e.g., 2A sequence derived from foot-and-mouth disease virus (FMDV) (F2A), 2A sequence derived from equine rhinitis A virus (ERAV) (E2A), 2A sequence derived from Porcine teschovirus (PTV-1) (P2A), 2A sequence derived from Thosea asigna virus (TaV) (T2A sequence) (PLoS ONE 3, e2532, 2008, Stem Cells 25, 1707, 2007)), internal ribosome entry site (IRES) (U.S. Patent No. 4,937,190) and the like.

As the promoters for the above-mentioned vector, used are EF1a promoter, CAG promoter, SRa promoter, SV40 promoter, LTR promoter, CMV (cytomegalovirus) promoter, RSV (Rous sarcoma virus) promoter, MoMuLV (Moloney mouse leukemia virus) LTR, HSV-TK (simple herpes virus thymidine kinase) promoter, TCR Va gene promoter, TCR Vβ gene promoter and the like.

When desired, the above-mentioned vector may contain, in addition to the above-mentioned promoters, transcription and translation regulating sequences, ribosome binding site, enhancer, replication origin, polyadenylation signal, selectable marker gene, and the like. Examples of the selectable marker gene include dihydrofolate reductase gene, neomycin resistance gene, puromycin resistance gene, and the like.

(Host cell containing nucleic acid encoding anti-human TIGIT agonistic antibody)

In one embodiment of the present invention, an antibody can be formed in a host cell by introducing an expression vector containing a nucleic acid encoding the heavy chain and a nucleic acid encoding the light chain of the above-mentioned nucleic acid of the present invention into the host cell. Therefore, in one embodiment of the present invention, a host cell containing the nucleic acid or vector of the present invention (hereinafter sometimes referred to as "the host cell of the present invention") is provided. Furthermore, a production method of the antibody of the present invention by using the host cell (hereinafter sometimes to be referred to as "the production method of the present invention") is provided. The production method of the present invention includes a step of culturing the host cell of the present invention under conditions where the antibody of the present invention is produced.

Examples of cells that can be used as cells for expressing the antibody of the present invention or anti-human TIGIT agonistic antibody include mammalian cells. Such mammalian cells include, for example, many immortalized cell lines available from the American Type Culture Collection (ATCC), Manassas, VA. Examples thereof include, but are not limited to, Chinese hamster ovary (CHO) cell, HEK 293 cell, NSO cell, HeLa cell, baby hamster kidney (BHK) cell, monkey kidney cell (COS), human hepatocarcinoma cells (e.g., HepG2), and many other cell lines. Non-mammalian cells, including but not limited to bacteria (e.g., *Escherichia coli,* etc.), yeasts, insects, and plants, can also be used to express recombinant antibodies. Anti-human TIGIT agonistic antibodies can also be produced in transgenic animals such as cow and chicken.

The method for introducing a nucleic acid encoding an antibody or a fragment thereof (e.g., the nucleic acid of the present invention) or vector into the host cell is not particularly limited, and a known method can be used. When a nucleic acid or a plasmid vector is introduced, for example, calcium phosphate coprecipitation method, PEG method, electroporation method, microinjection method, lipofection method, or the like can be performed. A nucleic acid encoding an antibody or a fragment thereof, in the form of an RNA, may also be directly introduced into cells and used to express the antibody in the cells. As an introduction method of RNA, a known method can be used and, for example, lipofection method, electroporation method, or the like can be preferably used.

(Production method of anti-human TIGIT agonistic antibody)

The obtained recombinant host cell can be maintained under conditions suitable for expression (e.g., in the presence of inducer, in suitable non-human animal, medium added with suitable salt, growth factor, antibiotic, nutritional supplement and the like, and the like), whereby one or more polypeptides encoded thereby can be produced. In some cases, the heavy chain is produced with one cell, and the light chain is produced with another cell.

When a mammalian cell is used as a cell that expresses the antibody of the present invention, a minimum essential medium (MEM) [Science, 122,501 (1952)], Dulbecco's modified Eagle medium (DMEM) [Virology, 8,396 (1959)], RPMI 1640 medium [The Journal of the American Medical Association,199,519 (1967)], 199 medium [Proceeding of the Society for the Biological Medicine,73,1 (1950)] containing about 5 to about 20% fetal bovine serum, or the like is used as a medium for culturing the cells. The pH of the medium is preferably about 6 to about 8. Culture is generally performed at about 30°C to about 40°C. Aeration and stirring may be performed as necessary.

Reagent or kit for detection or measurement of human TIGIT

As mentioned above, the antibody of the present invention has a binding ability to human TIGIT. Therefore, in another embodiment, a reagent for detection or measurement of human TIGIT (or TIGIT other than human) containing the antibody of the present invention (hereinafter sometimes referred to as "the reagent of the present invention") is provided. The antibody of the present invention can be bound to a labeling substance or the like and the resulting antibody itself can also be used as a reagent for detecting or measuring human TIGIT, or it can be combined with other reagents and used to detect or measure human TIGIT. Therefore, a kit for detection or measurement of human TIGIT (hereinafter sometimes referred to as "the kit of the present invention") is also provided. The reagent or kit of the present invention can be used in methods using Western blotting, immunostaining, EIA (Enzyme Immunoassay), or ELISA (Enzyme-Linked immunosorbent assay) (e.g., direct method, indirect method, competitive method, sandwich method, etc.).

The reagent or kit of the present invention may contain the antibody of the present invention, as well as other antibody (including a fragment thereof, hereinafter the same) or a reagent, and the like. The antibody or reagent and the like may be blended in advance with the above-mentioned antibody, or may be stored in separate containers. Examples of the antibody or reagent and the like include an antibody or a fragment thereof which is different from the antibody of the present invention that binds to human TIGIT (hereinafter sometimes referred to as "anti-human TIGIT antibody"), secondary antibody, labeling substance (e.g., fluorescent dye (e.g., fluorescein, etc.), enzyme (e.g., Horse radish peroxidase: HRP, alkali phosphatase (AP), etc.), radioactive substance, and the like), substrate (AP substrate PNPP (p-nitrophenylphosphate), HRP substrate ABTS (2,2'-Azinobis[3-ethylbenzothiazoline-6-sulfonic acid]-diammonium salt), OPD (o-phenylenediamine dihydrochloride), TMB (3,3',5,5'-tetramethylbenzidine), etc.), stop solution for stopping an enzyme reaction, carrier, human TIGIT conjugated with a labeling substance (hereinafter sometimes referred to as "labeled human TIGIT"), reaction vessel, buffer for diluting treated solution and antibodies, reference material with known concentration, positive control (e.g., recombinant human TIGIT, etc.), negative control, manufacturer's instructions describing protocol, and the like. These reagents and the like can be mixed in advance as necessary.

The antibody of the present invention to be contained in the above-mentioned reagent or kit may be immobilized on a carrier in advance. The carrier to be used in present invention is not particularly limited and includes, for example, insoluble carriers such as polymers such as polystyrene and the like, glass bead, magnetic particles, microplate, filter paper for immunochromatography, glass filter, and the like. It is preferably a microplate used for ELISA. Also, human TIGIT (including human TIGIT conjugated with other peptide such as labeled human TIGIT, BSA, and the like) may be immobilized with the above-mentioned carrier (preferably, microplate). Therefore, in one embodiment of the present invention, a reagent or kit for ELISA containing a microplate on which the antibody of the present invention is immobilized, or a reagent or kit for ELISA containing a microplate on which human TIGIT is immobilized and the antibody of the present invention is provided.

The reagent or kit of the present invention containing a secondary antibody can be used for, for example, Western blotting, immunostaining, an indirect method of EIA or ELISA, and the like. The secondary antibody is not particularly limited as long as it can bind to the antibody of the present invention, and can be selected as appropriate. In addition, the secondary antibody is preferably bound with the above-mentioned labeling substance, and the above-mentioned reagent or kit may also contain the above-mentioned substrate for the labeling substance and the like. When the reagent or kit of the present invention is used for an indirect ELISA method, for example, human TIGIT is immobilized on a microplate, reacted with the antibody of the present invention and subsequently with an enzyme-labeled secondary antibody against the antibody, and washed. Then, the enzyme activity remaining in the microplate is detected or measured, whereby human TIGIT or its amount can be detected or measured.

The reagent or kit of the present invention containing labeled human TIGIT can be used, for example, in EIA or ELISA competitive method and the like. Labeled human TIGIT is not particularly limited as long as it can bind to the antibody of the present invention. The labeling substance to be bound to human TIGIT is not particularly limited, and examples thereof include the above-mentioned fluorescent dyes, enzymes, and the like. The labeling substance may be bound directly or indirectly to human TIGIT. Direct binding is performed by, for example, a crosslinking reaction via a linker (e.g., NHS ester, maleimide, etc.), but is not limited to this method. Indirect binding is performed by, for example, binding one or more tags (e.g., biotin, etc.) to biotin and binding the tag to a labeling substance via a substance that binds to the tag (e.g., (strept) avidin, an antibody against the tag, etc.). Therefore, labeled human TIGIT may be provided in a form in which the tag-added human TIGIT and a labeling substance having a substance that binds to the tag are different substances. The above-mentioned reagent or kit may contain, in addition to the labeled human TIGIT, the above-mentioned substrate and the like for the labeling substance. When the reagent or kit of the present invention is used in a competitive EIA or ELISA method, for example, the antibody of the present invention is immobilized on a microplate, and a sample containing human TIGIT and a labeled human TIGIT with a known concentration are simultaneously reacted in the same microplate, and the enzyme activity remaining in the microplate after the reaction is detected or measured, whereby human TIGIT or its amount can be detected or measured.

The reagent or kit of the present invention containing anti-human TIGIT can be used, for example, in an ELISA sandwich method and the like. The antibody that binds to human TIGIT is not particularly limited as long as it can bind to human TIGIT, but it is preferably bound with a labeling substance. Such labeling substance is not particularly limited, and examples thereof include the above-mentioned fluorescent dyes, enzymes, and the like. The above-mentioned reagent or kit may contain the above-mentioned substrate for the labeling substance and the like, in addition to the anti-human TIGIT antibody. When the reagent or kit of the present invention is used in the ELISA sandwich method, for example, the antibody of the present invention is immobilized on a microplate, reacted with human TIGIT, and subsequently with a labeled anti-human TIGIT antibody, and washed. Then, the enzyme activity remaining in the microplate is detected or measured, whereby human TIGIT can be detected or measured.

Anti-human TIGIT antibody can be produced by existing general production methods and using, as immunogen, human TIGIT, a partial peptide thereof, or modified human TIGIT with amino acid, etc. added thereto. Such antibody includes, but is not limited to, polyclonal antibody, monoclonal antibody (mAb), fragment thereof, or the like. Preferred antibody is a monoclonal antibody or a fragment thereof. Examples of the antibody or fragment thereof include, but are not limited to, genetically engineered conjugate molecules such as fragments (e.g., Fab, Fab', F(ab')2, etc.) which have the ability to bind to human TIGIT, or derivatives thereof modified with polyethylene glycol (PEG) and the like having a protein stabilizing action. Alternatively, a commercially available antibody may also be used as the above-mentioned anti-human TIGIT antibody.

Method for detection or measurement of human TIGIT

In another embodiment, the present invention provides a method for detecting or measuring human TIGIT by using the antibody, the reagent, or the kit of the present invention (hereinafter sometimes referred to as "the method of the present invention"). In one embodiment, the method of the present invention includes

(1) a step of contacting human TIGIT in a sample with the antibody of the present invention, and
(2) a step of detecting or measuring the antibody of the present invention bound to the human TIGIT.

The aforementioned method can be applied, for example, to Western blotting, immunostaining, EIA or ELISA (e.g., direct method, indirect method, sandwich method, etc.), and the like.

In the above-mentioned step (1), the method of contacting the human TIGIT in the sample with the antibody of the present invention is not particularly limited and, for example, the sample may be mixed with a solution containing the antibody of the present invention. When the sample is a tissue section, human TIGIT and the antibody of the present invention can also be contacted by adding a solution containing the antibody of the present invention to the tissue section. The conditions for contacting human TIGIT with the antibody of the present invention are not particularly limited. They are generally contacted at a temperature of 0 to 45°C, preferably 0 to 40°C, more preferably 4 to 37°C, further preferably 25°C to 37°C. The contact time is not particularly limited, and it is generally 5 min to 6 hr, preferably 10 min to 2 hr, more preferably 20 min to 1 hr.

The above-mentioned step (1) can be performed by adding the sample to the carrier on which the antibody of the present invention is immobilized, or immobilizing the human TIGIT in the sample on the carrier and adding the antibody of the present invention to the carrier. As such carrier, those similar to the ones described in the above-mentioned 1. can be used. The method for immobilizing the antibody of the present invention or the human TIGIT in the sample on a carrier is not particularly limited. For example, methods using physical adsorption, electrostatic interaction, hydrophobic interaction, crosslinking agents, and the like can be mentioned. The concentration of the antibody of the present invention in the solution during immobilization may be appropriately adjusted according to the material and shape of the carrier, immobilization method, and the like. It is, for example, 5 to 50 μg/mL, preferably 10 to 40 μg/mL. When immobilizing human TIGIT, the concentration of human TIGIT in the solution can be adjusted as appropriate. A blocking operation on the immobilized carrier with a blocking agent suppresses nonspecific adsorption of the antibody of the present invention or human TIGIT and also suppresses the background during measurement. The blocking agent may be appropriately selected from agents generally used in the field of measurement using an antigen-antibody reaction such as EIA and ELISA, and a preferred blocking agent is collagen peptide.

After step (1), a washing step may be provided to remove antibodies that could not bind to human TIGIT or human TIGIT that could not bind to antibodies. Examples of the washing solution to be used in the washing step include buffers (pH 6 to 8), more specifically Tris buffer, phosphate buffer, HEPES buffer, and the like. In addition, these buffers may contain salt, surfactant, protein, sugar, amphoteric ion compound and the like as appropriate. In addition, washing solutions contained in commercially available kits for immune response are also used preferably.

The method for detecting or measuring the antibody of the present invention bound to human TIGIT in the above-mentioned step (2) is not particularly limited, and can be performed by a method known per se. Examples of such method include the methods described in "Methods in ENZYMOLOGY" Vol.70 (Immunochemical Techniques (Part A)), ibidem Vol.73 (Immunochemical Techniques (Part B)), ibidem Vol.74 (Immunochemical Techniques (Part C)), ibidem Vol.84 (Immunochemical Techniques (Part D:Selected Immunoassays)), ibidem Vol.92 (Immunochemical Techniques (Part E:Monoclonal Antibodies and General Immunoassay Methods)), ibidem Vol.121 (Immunochemical Techniques (Part I:Hybridoma Technology and Monoclonal Antibodies)) (all published by Academic Press), and the like.

Specifically, for example, when the antibody of the present invention conjugated with a labeling substance is used in the above-mentioned step (1), the antibody of the present invention conjugated with human TIGIT or its amount can be detected or measured by detecting or measuring the labeling substance or its amount. Alternatively, after step (2), a complex of the antibody of the present invention and human TIGIT is contacted with a secondary antibody conjugated with a labeling substance and bound to the antibody of the present invention, or an anti-human TIGIT antibody conjugated with a labeling substance, and the labeling substance or its amount may be detected or measured. Prior to detection or measurement, a washing step may be provided to remove secondary antibody or anti-human TIGIT antibody that has failed to bind to the antibody of the present invention or human TIGIT. The washing step can be performed by the same method as the washing step optionally performed after the aforementioned step (1). As the secondary antibody and the anti-human TIGIT antibody, those similar to the ones described in above-mentioned 3 can be used.

When a fluorescent substance is used as the labeling substance, the labeling substance or its amount can be detected or measured by measuring the fluorescence emitted by the labeling substance. When an enzyme is used as a labeling substance, a substrate that is decomposed by the action of the enzyme to develop color, luminescence, or the like is added, and the color development, luminescence, the level thereof, etc. can be detected or measured using a plate reader or the like. Moreover, when a radioactive substance is used as the labeling substance, the radiation dose emitted by the substance can be detected or measured using a scintillation counter or the like. Based on the data thus detected or measured, the amount of human TIGIT can be quantified using image processing software (e.g., Image J, etc.) and the like.

In another embodiment of the method of the present invention, the method of the present invention includes

(1') a step of contacting labeled human TIGIT and human TIGIT in a sample with the antibody of the present invention, and
(2') a step of detecting or measuring the antibody of the present invention bound to the labeled human TIGIT.

The aforementioned method can be applied, for example, to EIA or ELISA competitive method and the like.

In the above-mentioned step (1'), the method of contacting the human TIGIT in the sample with the antibody of the present invention is not particularly limited and, for example, a solution containing labeled human TIGIT, the sample, and a solution containing the antibody of the present invention may be mixed. The conditions for contacting labeled human TIGIT and human TIGIT, with the antibody of the present invention are, for example, those similar to the conditions described in the above-mentioned step (1).

The above-mentioned step (1') can be performed by adding the sample and labeled human TIGIT with a known concentration to the carrier on which the antibody of the present invention is immobilized, or immobilizing the human TIGIT in the sample on the carrier and adding labeled human TIGIT and the antibody of the present invention to the carrier. As the kind of such carrier, immobilization method, blocking agent and the like, those similar to the ones described in the above-mentioned step (1) can be used.

After step (1'), a washing step may be provided to remove antibodies that could not bind to human TIGIT or human TIGIT or labeled human TIGIT that could not bind to antibodies. Examples of the washing solution to be used in the washing step include those similar to the ones described in the above-mentioned step (1).

The method for detecting or measuring the antibody of the present invention bound to labeled human TIGIT in the above-mentioned step (2') can detect or measure the antibody of the present invention conjugated with human TIGIT or its amount by detecting or measuring the labeling substance conjugated with labeled human TIGIT or its amount.

When a fluorescent substance is used as the labeling substance, the labeling substance or its amount can be detected or measured by measuring the fluorescence emitted by the labeling substance. When an enzyme is used as a labeling substance, a substrate that is decomposed by the action of the enzyme to develop color, luminescence, or the like is added, and the color development, luminescence, the level thereof, etc. can be detected or measured using a plate reader or the like. Moreover, when a radioactive substance is used as the labeling substance, the radiation dose emitted by the substance can be detected or measured using a scintillation counter or the like. In the method of the present invention, when the sample contains a large amount of human TIGIT, the amount of labeled human TIGIT that can bind to the antibody of the present invention decreases, resulting in a weaker labeling level (e.g., color development or fluorescence level). On the other hand, when the amount of human TIGIT in the sample is low, the amount of labeled human TIGIT that can bind to the antibody of the present invention increases, resulting in a stronger labeling level (e.g., color development or fluorescence level).

Examples of the samples used in the method of the present invention include samples collected from animals. The aforementioned sample may be a sample known to contain human TIGIT (for example, containing the morphology, etc, of cells expressing human TIGIT), or a sample unknown as to whether it contains human TIGIT. Examples of such animal include mammals such as mouse, rat, hamster, guinea pig, dog, monkey, orangutan, chimpanzee, human, and the like. Examples of the animal-derived sample include blood, serum, plasma, saliva, urine, tear, sweat, milk, nasal discharge, semen, pleural effusion, gastrointestinal secretion, cerebrospinal fluid, interstitial fluid, and lymph, preferably serum and plasma. A cell population obtained by culturing cells is also preferred. These samples can be obtained by a method known per se. For example, serum and plasma can be prepared by collecting blood from a subject animal according to a conventional method and separating the liquid component, and the cerebrospinal fluid can be collected by a known means such as spinal tap and the like.

Medicament containing the antibody of the present invention

In addition, the present invention provides a medicament containing the above-mentioned antibody of the present invention as an active ingredient. The antibody of the present invention can suppress immune activation via TIGIT activation in cytotoxic T cells (CTL) such as CD8-positive T cells or natural killer (NK) cells, and can also enhance the ability to suppress immune responses via TIGIT activation in regulatory T cells (Treg cells). Therefore, a medicament containing the antibody of the present invention can be used for the prophylaxis or treatment of autoimmune diseases and diseases associated with the activation of CTL or NK cell. Examples of such disease include rheumatoid arthritis, psoriasis, psoriatic arthritis, multiple sclerosis (MS), inflammatory bowel disease (IBD), celiac disease, graft-versus-host disease (GVHD), and irritable bowel syndrome. The above-mentioned diseases also include, for example, Sjogren's syndrome, non-alcoholic steatohepatitis (NASH), and primary biliary cirrhosis. The subject of administration of the medicament of the present invention includes primates, including lemur, loris, tupai, monkey (e.g., *Macaca fascicularis,* etc.), human, preferably human.

The antibody of the present invention and an anti-human TIGIT agonistic antibody are preferably prepared as pharmaceutical compositions according to a conventional method. Where necessary, moreover, the therapeutic agent of the present invention may contain a pharmaceutically acceptable carrier and/or additive. For example, it can contain surfactant (PEG, Tween etc.), excipient, antioxidant (ascorbic acid etc.), colorant, flavor, preservative, stabilizer, buffering agent (phosphate, citrate, other organic acid etc.), chelating agent (EDTA etc.), suspending agent, isotonizing agent, binder, disintegrant, lubricant, glidant, corrigent and the like. Not being limited to these, the above-mentioned pharmaceutical composition may contain other conventional carriers as appropriate. Specific examples include light anhydrous silicic acid, lactose, crystalline cellulose, mannitol, starch, carmellose calcium, carmellose sodium, hydroxypropylcellulose, hydroxypropylmethylcellulose, polyvinyl acetaldiethylaminoacetate, polyvinylpyrrolidone, gelatin, medium-chain fatty acid triglyceride, polyoxyethylene hydrogenated castor oil 60, sucrose, carboxymethylcellulose, cornstarch, inorganic salts and the like. It may also contain other low-molecular-weight polypeptide, proteins such as serum albumin, gelatin and immunoglobulin and the like, as well as amino acid. When an aqueous solution for injection is formulated, the anti-human TIGIT agonistic antibody is dissolved in, for example, isotonic solution containing saline, glucose or other auxiliary agent. Examples of the auxiliary agent include D-sorbitol, D-mannose, D-mannitol, and sodium chloride, and may be used in combination with suitable solubilizing agents, for example, alcohol (ethanol etc.), polyalcohol (propylene glycol, PEG etc.), non-ionic surfactant (polysorbate80, HCO-50) and the like.

Where necessary, polypeptide may also be encapsulated in a microcapsule (microcapsules made of hydroxymethylcellulose, gelatin, poly[methylmethacrylate] and the like), or formulated as a colloid drug delivery system (liposome, albumin microsphere, microemulsion, nanoparticle, nanocapsule, etc.) (see Remington's Pharmaceutical Science 16th edition &, Oslo Ed. (1980) etc.).

The content of the anti-human TIGIT agonistic antibody in a pharmaceutical composition is, for example, about 0.01 - 100 wt%, preferably 0.1 - 99.9 wt%, of the whole pharmaceutical composition.

While the above-mentioned pharmaceutical composition can be administered both orally and parenterally, it is preferably administered parenterally. Specifically, it is administered to patients by injection or transdermal administration. As an example of the dosage form of injection, it can be administered systemically or topically by intravenously injection, intramuscular injection, subcutaneous injection and the like. It may also be administered to the treatment site or in the vicinity thereof by topical injection, particularly intramuscular injection. Examples of the dosage form of transdermal administration include ointment, gel, cream, plaster, patch and the like, which can be administered systemically or topically. In addition, the administration method can be appropriately selected according to the age and symptom of the patients. The dose can be selected from, for example, the range of 0.5 mg - 10 mg/kg body weight as the anti-human TIGIT agonistic antibody. However, the pharmaceutical composition of the present invention is not limited by these doses.

The SEQ ID NOs: of the Sequence Listing in the present specification show the following sequences. (SEQ ID NO: 1) human TIGIT amino acid sequence (SEQ ID NO: 2) Clone M1-8 H-CDR1 amino acid sequence (SEQ ID NO: 3) Clone M1-8 H-CDR2 amino acid sequence (SEQ ID NO: 4) Clone M1-8 H-CDR3 amino acid sequence (SEQ ID NO: 5) Clone M1-8 L-CDR1 amino acid sequence (SEQ ID NO: 6) Clone M1-8 L-CDR3 amino acid sequence (SEQ ID NO: 7) Clone M1-8 heavy chain variable region amino acid sequence (mouse heavy chain variable region) (SEQ ID NO: 8) Clone M1-8 light chain variable region amino acid sequence (mouse light chain variable region)

While the present invention is more specifically explained by the following Examples, the scope of the present invention is not limited to those Examples.

[Example]

Example 1: Production of novel anti-human TIGIT agonistic antibody (M1-8)

<Production of recombinant human TIGIT-His-mFc protein>

A recombinant human TIGIT-His-mFc protein was prepared using Expi293 Expression System (Thermo). Cells transfected with a human TIGIT-His-mFc expression plasmid were cultured, then the recombinant human TIGIT-His-mFc protein was purified from its culture supernatant by gel filtration using Ni-NTA cartridge (FUJIFILM Wako Pure Chemical Corporation) column and Superdex200 pg column.

<Immunization method>

The recombinant human TIGIT-His-mFc protein was subcutaneously immunized to both popliteal fossa of 6- to 8-week-old female CD2F1 mice at 10 μg/mouse, together with TiterMax. Thereafter, the mice were booster immunized with the same amount of the immunogen together with CpG/Alum 11 times every 4-5 days. Three days before isolating lymph nodes, the immunogen diluted to 1 mg/mL with phosphate buffered saline (PBS, pH 7.4) was administered as the final immunization.

<Measurement of blood antibody titer>

At 36 days after the initial immunization, blood sample was collected from tail vein, and the obtained blood was diluted with physiological saline by 100 times. The diluted blood was centrifuged at 4°C for 5 min, and the supernatant was recovered to give an antiserum. The antibody titer was determined by the following method. Doxycycline-expression inducible human TIGIT-expressing cells were cultured for 2 days in the presence or absence of doxycycline. The cells were washed with PBS, then Cell Dissociation buffer (GIBCO) was added, and the cells were dissociated from flask by standing at room temperature for 10 min. The obtained cells were washed twice with FACS buffer (PBS containing 1% FBS), and respectively suspended using FACS buffer at the cell density of $1 \times 10^6$ cells/mL. The suspension was dispensed by 100 μL in a 96-well V-bottom plate, centrifuged, and the supernatant was removed. To this plate, antiserum diluted with FACS buffer was dispensed by 100 μL, and the cells were allowed to stand at 4°C for 30 min. After washing 3 times with FACS buffer, AlexaFluoro647 (registered trademark)-labeled anti-mouse IgG antibody was dispensed by 100 μL, and the cells were allowed to stand at 4°C for 30 min. After washing 2 times with FACS buffer, the cells were suspended in 200 μL of FACS buffer, and the antibody bound thereto was measured using Cytomics FC500 MPL.

Fig. 1 shows the obtained Median fluorescence intensity. An increase in the antibody titer against doxycycline-expression induced human TIGIT-expressing cells was found in the antisera of mice immunized with a recombinant TIGIT protein.

<Production of monoclonal antibody>

Three days after the final immunization, lymph nodes were excised from mice Nos. 1, 2, 4, and 5 in which an increase in antibody titer was observed, compressed on and filtered through a 40 um strainer, and suspended in DMEM. P3-X63.Ag8.U1 (P3U1) was used as a fusion partner for hybridoma production. Lymphocytes and P3U1 were each washed three times with DMEM and mixed at a cell number ratio of 1:1. Cells washed twice with fusion buffer (0.3 M mannitol, 0.1 mM calcium chloride, 0.1 mM magnesium sulphate) were suspended in fusion buffer at $1.51 \times 10^7$ cells/mL. Cell fusion was performed according to the manual using BTX. The fused cells were washed once with ClonalCell™-HY Medium C (STEMCELL), suspended in the same medium at $1.67 \times 10^6$ cells/mL, and allowed to stand overnight under 37°C, 5% $CO_2$ conditions. The obtained fused cells were centrifuged, suspended in ClonaCell™-HY Medium C at $1 \times 10^6$ cells/mL, mixed with ClonaCell™-HY medium D, and plated. After culturing for 10 days under 37°C, 5% $CO_2$ conditions, hybridoma colonies were picked using Clone Pix and seeded onto a plate to which ClonaCell™-HY medium E was added at 200 μL/well. After culturing for 3 days under 37°C, 5% $CO_2$ conditions, the culture supernatant was collected and subjected to screening.

<Production of transiently expressing cell>

Transiently expressing cells were prepared using the Expi293F expression system (Thermo) and according to its manual. Specifically, Expi293F cells in logarithmic growth phase were suspended at a cell density of $1 \times 10^6$ cells/mL, and dispensed by 35 mL to 125 mL Myer flasks. 37.5 μg of TIGIT expression plasmid and 50 μL of 293Fectin were each diluted in OptiMEM I medium to a final volume of 1.25 mL and allowed to stand at room temperature for 5 min. The diluted DNA solution and 293Fectin solution were mixed and allowed to stand at room temperature for 20 min. The mixture was added to the cells and cultured at 37°C, 8% $CO_2$, 120 rpm for 2 days, and the cells were collected.

<Primary screening (Mirrorball assay)>

Primary screening was performed using the transiently expressing cell line and its parent cell line. After harvesting the cells, the cells were washed three times with FACS buffer and suspended therein at $3 \times 10^5$ cells/mL. AlexaFluor (registered trademark) 647-labeled anti-mouse IgG (subclass 1+2a+2b+3) antibody was diluted with the same buffer at 1 μg/mL. Equal amounts of the cells and the antibody were mixed, and 20 μL each was dispensed into a 384-well plate. 10 μL of hybridoma culture supernatant diluted 10-fold with FACS buffer was added to the plate, stirred with a plate mixer, and centrifuged for 1 min. After allowing to stand at room temperature for 1 hr, the fluorescence intensity was measured using a plate imager (Mirrorball).

<Determination of antibody concentration in culture supernatant by ELISA method>

In a 96-half well ELISA plate, anti-mouse IgG (subclass 1+2a+2b+3) antibody diluted with PBS to 5 μg/mL was dispensed by 50 μL, and allowed to stand overnight at 4°C. Then, the plate was washed twice with a washing buffer (PBS containing 0.05% tween 20), a blocking buffer (PBS containing 20% immunoblock) was dispensed by 100 μL, and the mixture was allowed to stand at room temperature for 1 hr. To this plate, a hybridoma culture supernatant diluted 100-fold or 1,000-fold with an assay buffer (PBS containing 10% immunoblock) or a mouse IgG1 standard solution (0-300 ng/mL) was added by 50 μL, and the mixture was allowed to stand at room temperature for 1 hr. The plate was washed 3 times with a washing buffer, HRP-labeled anti-mouse IgG (subclass 1+2a+2b+3) antibody was dispensed by 50 μL, and the mixture was allowed to stand at room temperature for 1 hr. After washing 3 times with the washing buffer, Sure Blue/TMB Peroxidase Substrate (KPL) was dispensed by 50 μL and the mixture was allowed to react at room temperature for 5 min. 0.5 M sulfuric acid was dispensed by 50 μL to stop the reaction, and the absorbance at 450 nm was measured using a plate reader (SpectraMax). Softmax was used to form a calibration curve and calculate the concentration.

Human, monkey, and mouse TIGIT-expressing cells were generated using a doxycycline-expression inducible CHO cell line. That is, human, monkey, and mouse TIGIT expression plasmids were introduced into doxycycline-expression inducible CHO cell line. The transfected cells were cloned by the limiting dilution method and cultured in a doxycycline-containing medium to induce TIGIT expression, and cells in which expression was confirmed were selected as TIGIT-expressing cells.

<Secondary screening (FACS)>

Secondary screening was performed by a method similar to that in the blood antibody titer measurement. That is, doxycycline-expression inducible human TIGIT-expressing cells were cultured for 2 days in the presence or absence of doxycycline. The cells were washed with PBS, then Cell Dissociation buffer (GIBCO) was added, and the cells were dissociated from flask by standing at room temperature for 10 min. The obtained cells were washed twice with FACS buffer (PBS containing 1% FBS), and respectively suspended using FACS buffer at the cell density of $1 \times 10^6$ cells/mL. The suspension was dispensed by 100 $\mu$L in a 96-well V-bottom plate, centrifuged, and the supernatant was removed. To this plate, hybridoma culture supernatant diluted with FACS buffer to 1 $\mu$g/mL was added by 100 $\mu$L, and the cells were allowed to stand at 4°C for 30 min. After washing 3 times with FACS buffer, AlexaFluoro647 (registered trademark)-labeled anti-mouse IgG antibody was dispensed by 100 $\mu$L, and the cells were allowed to stand at 4°C for 30 min. After washing 2 times with FACS buffer, the cells were suspended in 200 $\mu$L of FACS buffer, and the antibody bound thereto was measured using Cytomics FC500 MPL.

Fig. 2 shows the binding activity of M1-8 antibody in the culture supernatant to doxycycline-expression induced human TIGIT-expressing cells.

<Tertiary screening (inhibitory assay for binding of recombinant TIGIT and PVR-expressing cell)>

Recombinant human TIGIT-human IgG1 Fc fused protein (R&D systems, 7898-TG-050) diluted to 2 nM with FACS buffer and hybridoma culture supernatant diluted to 10- to 1,000 ng/mL were mixed at 1:1 in a 96-well V-bottom plate, and the mixture was allowed to stand at room temperature for 30 min. Doxycycline-expression induced human PVR-expressing cells cultured for 2 days in the presence of doxycycline were suspended in FACS buffer at $1 \times 10^6$ cells/mL, and dispensed by 100 $\mu$L to another 96-well V-bottom plate. The plate containing the cells was centrifuged, and the supernatant was discarded. A TIGIT and culture supernatant mixture which was mixed in advance was added and the cells were allowed to stand at 4°C for 30 min. After washing 3 times with FACS buffer, AlexaFluoro647 (registered trademark)-labeled anti-human IgG antibody was dispensed by 100 $\mu$L, and the cells were allowed to stand at 4°C for 30 min. After washing 2 times with FACS buffer, the cells were suspended in 200 $\mu$L of FACS buffer, and the TIGIT-Fc bound thereto was measured using Cytomics FC500 MPL.

Fig. 3 shows the results of inhibitory assay for binding of recombinant TIGIT and PVR-expressing cell. A commercially available anti-human TIGIT antibody (clone MSBA43) inhibited the binding of TIGIT to PVR-expressing cells, but the M1-8 antibody in the culture supernatant did not inhibit the binding.

<Isotyping of antibody by ELISA method>

In a 96-half well ELISA plate, various anti-mouse isotype antibodies (ITM, BioLegend) diluted with PBS to 5 ug/mL were dispensed by 50 $\mu$L, and allowed to stand overnight at 4°C. Then, the plate was washed twice with a washing buffer (PBS containing 0.05% tween 20), SuperBlock (PBS) Blocking Buffer (Thermo) was dispensed by 100 $\mu$L, and the mixture was allowed to stand at room temperature for 1 hr. To this plate, a hybridoma culture supernatant diluted 10-fold with an assay buffer (PBS containing 10% SuperBlock (PBS) Blocking Buffer) was added by 50 $\mu$L, and the mixture was allowed to stand at room temperature for 1 hr. The plate was washed 3 times with a washing buffer, HRP-labeled anti-mouse IgG antibody was dispensed by 50 $\mu$L, and the mixture was allowed to stand at room temperature for 1 hr. After washing 3 times with the washing buffer, Sure Blue/TMB Peroxidase Substrate (KPL) was dispensed by 50 $\mu$L and the mixture was allowed to react at room temperature for 5 min. 0.5 M sulfuric acid was dispensed by 50 $\mu$L to stop the reaction, and the absorbance at 450 nm was measured using a plate reader (SpectraMax). Isotyping revealed that the M1-8 antibody was mouse IgG1/k.

<Identification of antibody CDR regions>

M1-8 antibody CDRs were defined according to the IMGT numbering system.

<Antibody purification>

The cells were cultured in ClonaCell™-HY Medium E, 0.5 mL of hybridoma suspension in the logarithmic growth phase was added to a 24-well plate containing 0.5 mL of Daigo's T medium (10% Ultra Low IgG FBS, 1% MEM NEAA, 1% Sodium Pyruvate, 1% L-Alanyl-L-Glutamine, 1% Penicillin-Streptomycin, 43% F-12 nutrient mixture, 43% Iscove's Modified Dulbecco's medium), and the cells were cultured under 37°C, 5% $CO_2$ conditions. After culturing for 2 to 3 days, the cells were added to a 6-well plate containing 4 mL of Daigo's T medium and further cultured for 2 to 3 days to naturalize the cells to the Daigo's T medium. After confirming that the cells were sufficiently naturalized, they were added to a T-75 flask containing 45 mL of Daigo's T and cultured under 37°C, 5% $CO_2$ conditions. After 7 to 10 days, the culture medium was collected and centrifuged to obtain the culture supernatant.

The monoclonal antibody was purified from the obtained culture supernatant by protein A resin. 0.6 mL of 50% slurry protein A Sepharose resin equilibrated with PBS was added to the culture supernatant and the mixture was shaken overnight at 4°C. After centrifugation at 4°C for 10 min, the supernatant was partially removed by suction, and the resin was suspended in the remaining medium and added to a 24-well filter plate (Whatman). After washing the resin three times with 5 mL of PBS, the bound antibody was eluted with 2.5 mL of elution buffer (0.1 M Glycine-HCl/0.3 M NaCl, pH 3.0). The eluate was immediately neutralized with 0.3 mL of neutralization buffer (1 M Tris-HCl, pH 8.0). Mouse IgG1 was purified by mixing an equal amount of the culture supernatant with mouse IgG1-binding buffer (1.5 M Glycine-HCl, 3 M NaCl, pH 9.5) and then adding protein A Sepharose resin. Also, a mouse IgG1-binding buffer was used instead of PBS during washing. The obtained purified antibody was buffer-substituted in PBS using an ultrafiltration column (Amicon), and the concentration was measured using a spectrophotometer (NanoDrop).

<Determination of biological activity of purified antibody>

· Preparation of serially diluted solutions of antibody (Day 0)

Test antibody and PVR (Recombinant Human CD155/PVR, R&D systems, 2530-CD-050) as a positive control, and IgG as a negative control were serially diluted with a medium (RPMI1640: FUJIFILM Wako Pure Chemical Corporation, 189-02025, 10% FBS: CORNING, 37-076-CVR, 1×PS: penicillin-streptomycin solution (×100), FUJIFILM Wako Pure Chemical Corporation, 168-23191). Serially diluted solutions were prepared for test antibody and IgG at 5 concentrations from final concentration 10 ug/mL to 100 ng/mL whose common ratio was 3, and PVR at 5 concentrations from final concentration 33 ug/mL to 333 ng/mL whose common ratio was 3.

·Preparation of coating plate (Day 0)

Anti-human CD3 (eBioscience, 16-0037) was diluted with PBS (D-PBS(-), FUJIFILM Wako Pure Chemical Corporation, 045-29795) to 3 µg/mL, and added to a 96-well plate (black solid flat-bottom cell culture surface-treated polystyrene microplate, CORNING, 3916) by 50 µL/well. It was sealed and allowed to stand overnight in a refrigerator at 4°C.

·Cell seeding and addition of sample (agonist assay) (Day 0)

Doxycycline was added to doxycycline-expression inducible human TIGIT-expressing cells (hTIGIT/Jurkat) at 2 µg/mL, and seeded at 60 µL/well in a 96-well plate (transparent flat-bottom cell culture surface-treated microplate, CORNING, 3598) containing a medium added in advance at 48 µL/well. A test antibody serially diluted in advance, a positive control, and a negative control were added at 12 µL/well. To a 100% control well was added PVR at a final concentration of 3.3 µg/mL, and a medium was added at 12 µL/well to a 0% control well. The cells were cultured overnight in a $CO_2$ incubator (37°C, 5% $CO_2$).

·Cell seeding and addition of sample (antagonist assay) (Day 0)

Doxycycline was added to doxycycline-expression inducible human TIGIT-expressing cells (hTIGIT/Jurkat) at 2 µg/mL, and seeded at 60 µL/well in a 96-well plate (transparent flat-bottom cell culture surface-treated microplate, CORNING, 3598) containing a medium added in advance at 36 µL/well for the wells of the sample, the positive control, and the negative control, and at 48 µL/well for the wells of the 100% control and 0% control. A test antibody serially diluted in advance, a positive control, and a negative control were added at 12 µL/well. To a 100% control well was added a medium and PVR at a final concentration of 3.3 µg/mL was added to the 0% control well at 12 µL/well. Furthermore, PVR at a final concentration of 3.3 µg/mL was added to the sample, positive control, and negative control wells at 12 µL/well. The cells were cultured overnight in a $CO_2$ incubator (37°C, 5% $CO_2$).

·Cell seeding and addition of sample (Cytotoxicity/Growth inhibition assay) (Day 0)

Doxycycline was added to doxycycline-expression inducible human TIGIT-expressing cells (hTIGIT/Jurkat) at 2 µg/mL, and seeded at 60 µL/well, except for the 0% control wells, in a 96-well plate (transparent flat-bottom cell culture surface-treated microplate, CORNING, 3598) containing a medium added in advance at 48 µL/well. To the 0% control wells was added a medium at 60 µL/well. Furthermore, a test antibody serially diluted in advance, a positive control, and a negative control were added at 12 µL/well. To a 100% control well was added PVR at a final concentration of 3.3 µg/mL, and a medium was added at 12 µL/well to a 0% control well. The cells were cultured overnight in a $CO_2$ incubator (37°C, 5% $CO_2$).

·Cell stimulation (Day 1)

The solution was removed from the anti-human CD3-coated plate. After washing once with 200 μL/well of PBS, 90 μL/well of the cell solution for each assay, to which the sample was added on Day 0, was transferred. Anti-human CD28 (eBioscience, 16-0289) was diluted to a final concentration of 2 ug/mL and added to the plate at 10 μL/well. After stirring in a plate mixer (Titramax 100, Heidolph), the cells were cultured overnight in a $CO_2$ incubator (37°C, 5% $CO_2$).

·Measurement (Day 2)

Nano-Glo (registered trademark) Luciferase Assay System (Promega, N1120) was used for the measurement of Agonist assay and Antagonist assay, and CellTiter-Glo (registered trademark) Luminescent Cell Viability Assay System (Promega, G7572) was used for the measurement of Cytotoxicity/Growth inhibition assay. After returning the cells to room temperature from the $CO_2$ incubator, 100 μL/well of Nano-Glo™ Luciferase Assay Substrate solution diluted 50-fold with Nano-Glo™ Luciferase Assay Buffer, or 100 μL/well of CellTiter-Glo (registered trademark) Substrate solution dissolved in CellTiter-Glo (registered trademark) Buffer was added to the cells. After stirring in a plate mixer (Titramax 100, Heidolph), the cells were incubated for 3 min, and then the luminescence value was measured for 1 sec with a multilabel counter Envision (2103 Envision, PerkinElmer).

·Analysis

The sample values were calculated by the following calculation formulas.
Agonist assay:

```
agonist activity (%) of sample = (1 - (measurement value of
compound - mean of 100% control well)/(mean of 0% control -
mean of 100% control well))×100
```

The calculated expression degree (%) was graphed using GraphPad Prism and EC50 value was calculated.
Antagonist assay:

```
antagonist activity (%) of sample = (measurement value of
compound - mean of 100% control well)/(mean of 0% control -
mean of 100% control well)×100
```

The calculated inhibitory rate (%) was graphed using GraphPad Prism and IC50 value was calculated.
Cytotoxicity/Growth inhibition assay:

```
cytotoxicity (%) of sample = (1 - (measurement value of
compound -mean of 0% control well)/(mean of 100% control - mean
of 0% control well))×100
```

The calculated toxicity (%) was graphed using GraphPad Prism and IC50 value was calculated.
The measurement results of the biological activity of M1-8 antibody are shown in Fig. 4. M1-8 antibody showed an agonist activity but did not show an antagonist activity. In addition, M1-8 antibody did not show a cytotoxic activity under the present conditions against the human TIGIT-expressing cells used for this measurement, and did not influence cellular proliferation.

<Cross-reactivity test>

Cross-reactivity test of the obtained antibody was performed by FACS using human, monkey, and mouse TIGIT-expressing cells. That is, doxycycline-expression inducible cells were cultured for 2 days in the presence or absence of doxycycline. The cells were washed with PBS, then Cell Dissociation buffer (GIBCO) was added, and the cells were dissociated from flask by standing at room temperature for 10 min. The obtained cells were washed twice with FACS buffer (PBS containing 1% FBS), and respectively suspended using FACS buffer at the cell density of $1 \times 10^6$ cells/mL. The suspension was dispensed by 100 $\mu$L in a 96-well V-bottom plate, centrifuged, and the supernatant was removed. To this plate, purified antibody diluted with FACS buffer to 1 $\mu$g/mL was added by 100 $\mu$L, and the cells were allowed to stand at 4°C for 30 min. After washing 3 times with FACS buffer, AlexaFluoro647 (registered trademark)-labeled anti-mouse IgG antibody was dispensed by 100 $\mu$L, and the cells were allowed to stand at 4°C for 30 min. After washing 2 times with FACS buffer, the cells were suspended in 200 $\mu$L of FACS buffer, and the antibody bound thereto was measured using Cytomics FC500 MPL.

The results of cross-reactivity of M1-8 antibody with human, monkey, and mouse TIGIT are shown in Fig. 5. It was found that antibody M1-8 binds to human and monkey TIGIT, but not to mouse TIGIT.

In the following Examples, the following antibodies were used:

Human; anti-CD3 (Hit3a), anti-CD4 (FITC, OKT4 or BV510, OKT4), anti-CD19 (BV421, HIB19), anti-CD25 (PE, BC96), anti-CD27 (BV510, O323), CD38 (FITC, HIT2) anti-CD45RA (BV421, HI100), anti-CD127 (FITC, A019D5), anti-CD138 (APC, MI15), anti-CXCR5 (PerCP/Cy5.5, J252D4), anti-human TIGIT (PE-Cy7, A15153G) (all from BioLegend), 7-aminoactinomycin D (Bay Bioscience), anti-TIGIT agonistic antibody (Clone M1-8, Takeda Pharmaceutical Company, Limited).

Mouse; surface staining: anti-CD44 (FITC, IM7), anti-CD62L (PE, MEL-14), anti-CD69 (APC, H.12F3), anti-CD95 (APC, SA367H8), anti-CD138 (BV421, 281-2), anti-CD185 (PE, L138D7), anti-GL7 (PE-Cy7, GL7), (all from BioLegend), anti-B220 (PE, RA3-6B2), anti-CD3ε (PE-cy5, 145-2C11), anti-CD4 (eFluor450 or APC (all from Thermo Fisher Scientific). intracellular staining: anti-Foxp3 (PE, FJK-16s Thermo Fisher Scientific).

Isotype control; mIgG1 (MG1-45) (Biolegend).

complete RPMI (RPMI1640 supplemented with the following: 2 mM L-glutamine, 1% non-essential amino acids, 55 $\mu$M 2-mercaptoethanol (Thermo Fisher Scientific), 1 mM sodium pyruvate, 100 U/ml penicillin, 100 U/ml streptomycin, 10% FBS (Thermo Fisher Scientific)).

In statistical analysis, difference between continuous variables was examined using Wilcoxon rank sum test. P value <0.05 was considered significant. All statistical analyses were performed using JMP 15 (SAS Institute).

Example 2: Verification of suppression of CD8-positive T cell activation via TIGIT activation

Human peripheral bloods were collected from 4 healthy subjects into heparin blood collection tubes (Terumo), and PBMCs were separated using Ficoll-paque plus (GE healthcare). CD8-positive T cells were isolated from PBMC by MACS using a CD8+ T cell isolation kit (Miltenyi). Those cells were stained with a CFSE (Invitrogen) reagent, suspended in RPMI1640/10% FBS/PSG medium, and seeded in a 96-well round-bottom plate at $1 \times 10^5$ cells/well. The seeded cells were mixed with anti-human CD3 antibody (Biolegend) and human PVR-Fc (R&D systems) or human IgG1-Fc (R&D systems) bound to Dynabeads-M450 (Invitrogen) to $1 \times 10^5$ beads/well and cultured for 3 days under 5% CO$_2$, 37°C conditions.

The method for binding antibody to Dynabeads-M450 was as follows. Dynabeads-M450 was suspended in a binding buffer (0.1 M sodium phosphate pH 7.6), washed once using a magnet, and suspended again in the binding buffer. The suspension was mixed with 2.5 $\mu$g/$10^7$ beads of anti-CD3 antibody, PVR-Fc, and IgG1-Fc such that the total of PVR-Fc and IgG1-Fc was 2.5 $\mu$g/$10^7$ beads, and the mixture was rotated overnight at 4°C. The next day, the cells were washed twice with a washing buffer (PBS/0.1% BSA/2 mM EDTA) and a magnet, and mixed with a blocking buffer (0.2 M Tris-HCl pH 8.5/0.1% BSA) by rotating the mixture at room temperature for 4 hr. The cells were washed once using a washing buffer and a magnet, and finally suspended in the washing buffer at $4 \times 10^7$ beads/ml. The prepared Dynabeads/antibody/Fc complex was stored at 4°C and used for subsequent experiments.

CD8-positive T cells and Dynabeads/anti-CD3 antibody/PVR-Fc were mixed and cultured for 3 days under 5% CO$_2$, 37°C conditions. The culture supernatant was collected and the production amounts of IFNγ (Biolegend), Granzyme B (Mabtech), and Perforin (Mabtech) were measured by the ELISA method. The cells were collected, stained with anti-CD3 antibody-APCCy7, anti-CD8 antibody-BV421, and anti-CD4 antibody-BV510 (all from Biolegend), detected with CFSE by FACS, and the proportion of cell proliferation was quantitatively analyzed based on the dilution ratio of CFSE. The results are shown in Fig. 6. As shown in Fig. 6, the proliferation of CD8-positive T cells and the production of cytokines (IFNγ, Granzyme B, Perforin) were suppressed in a PVR-Fc amount-dependent manner. These results suggest that PVR suppresses activation of CD8-positive T cells through binding to its ligand, TIGIT, and that anti-TIGIT agonistic antibody suppresses activation of CD8-positive T cells.

Example 3: Effect on Tfh cells

PBMC was obtained from the blood collected in heparin blood collection tube (TERUMO, Tokyo, Japan) using Lymphoprep (Axis-Shield). Furthermore, CD4+ T cells were negatively selected using CD4+ T Cell Isolation Kit (Miltenyi Biotec). The cell surface was stained using various antibodies, and the following respective cell populations were fractionated using FACSAria III flow cytometer. Tfh; CD45RA-CXCR5+, Naive T; CD45RA+CXCR5-, non-Tfh; CD45RA-CXCR5-.

The obtained CD4+ naive T cells or Tfh cells were seeded onto a 96-well U plate at $1\times10^4$/well, Dynabeads human T-Activator CD3/CD28 (Thermo Fisher Scientific) was added in the same amount as the cells, and culture was started. Culture of each cell was started in 4 wells under the same conditions, then TIGIT expression on the cells was analyzed using FACSVerse flow cytometer 24 hr, 48 hr, 72 hr, and 96 hr later. Cell proliferation was analyzed using FlowJo software version 10.4.2 (FlowJo, OR, USA).

As a result, as shown in Fig. 7-1a, stimulation of Tfh cells having high TIGIT expression further increased TIGIT expression, whereas stimulation of naive T cells having low TIGIT expression did not increase TIGIT expression.

Respective cells were stained with 2 $\mu$M CellTrace Violet (Thermo Fisher Scientific) at 37°C for 7 min, washed, and cultured for 96 hr in a 96-well flat-bottom plate immobilized with anti-CD3 antibody 2 $\mu$g/ml, anti-CD28 antibody 1 ug/ml, and Isotype (mouseIgG1) or anti-TIGIT agonistic antibody 10 ug/ml. Dead cells were removed by 7-aminoactinomycin D staining, and viable cells were analyzed using FACSVerse flow cytometer. Cell proliferation was analyzed using FlowJo software version 10.4.2 (FlowJo, OR, USA).

As a result, as shown in Fig. 7-1b, anti-TIGIT agonistic antibody stimulation remarkably suppressed the proliferation of Tfh cells, whereas no suppressing effect was observed on naive T cells. That is, the anti-TIGIT agonistic antibody was shown to have a selective proliferation-suppressive effect on cells with high TIGIT expression.

In addition to CD4+ naive T cells and Tfh cells, non-Tfh cells were also examined for TIGIT expression on the cells in the same manner as above. As a result, TIGIT expression in the non-Tfh cells was of an intermediate level between Tfh cells and naive T cells (Fig. 7-2a).

In addition, the proliferation suppressing effect of the anti-TIGIT agonistic antibody was compared between Tfh cells and non-Tfh cells by flow cytometry analysis using CellTrance violet in the same manner as above. As a result, it was found that the antibody exhibits a proliferation suppressing effect on non-Tfh cells, and a more selective suppressing effect on Tfh cells (Fig. 7-2b).

Example 4: Effect of Tfh cells on B cells

PBMC was obtained from the blood collected in heparin blood collection tube (TERUMO, Tokyo, Japan) using Lymphoprep (Axis-Shield). Furthermore, CD19 microbeads were used to positively select CD19+ cells. The cell surface was stained using various antibodies, and the following respective cell populations were fractionated using FACSAria III flow cytometer. Memory B; CD19+CD27+, Effector T; CD25-CD45RA- (Tfh cells were isolated by the method in Example 3).

Tfh cells were cultured on a 96-well U plate, onto which 10 $\mu$g/ml of isotype (mouse IgG1) or anti-TIGIT agonistic antibody was immobilized, at 37°C for 30 min. Memory B cells were added thereto, and cultured in a medium mixed with 0.2 $\mu$g/ml of Staphylococcal enterotoxin B (Sigma) for 1 week. Dead cells were removed by 7-aminoactinomycin D staining, and viable cells were analyzed using FACSVerse flow cytometer. The proportion of plasma cells was analyzed using FlowJo software version 10.4.2 (FlowJo, OR, USA), and IgG in the culture supernatant was measured using human IgG ELISA kit (Bethyl).

As a result, as shown in Fig. 8, the group co-cultured with Tfh cells stimulated with anti-TIGIT agonistic antibody suppressed proliferation of plasma cells and also suppressed production of IgG.

Example 5: Effect on Treg cells

PBMC was obtained from the blood collected in heparin blood collection tube (TERUMO, Tokyo, Japan) using Lymphoprep (Axis-Shield). Furthermore, CD4+ T cells were negatively selected using CD4+ T Cell Isolation Kit (Miltenyi Biotec). The cell surface was stained using various antibodies, and the following respective cell populations were fractionated using FACSAria III flow cytometer (after lapse of 96 hours for Effector). Treg (FrI); CD25+CD45RA+, Treg (FrII); CD25+CD45RA-, Effector T; CD25-CD45RA-, Non-Treg responder CD4+ T cells; CD25-.

The obtained Treg cells (FrI) were cultured for 6 hr in a medium supplemented with IL-2 (R&D) 10 ng/ml in a 96-well flat-bottom plate immobilized with anti-CD3 antibody 2 $\mu$g/ml, anti-CD28 antibody 1 ug/ml, and Isotype (mouseIgG1) or anti-TIGIT agonistic antibody 10 ug/ml. On the other hand, the CD4+ Effector T cells fractionated after lapse of 96 hr were stained with CellTrace violet (CTV; Thermo Fisher Scientific) using a method similar to that in Example 2, washed, and cultured for 96 hr with activation-treated Treg cells at 1:1 ($1\times10^4$ each). Dead cells were removed by 7-aminoactinomycin D staining, and viable cells were analyzed using FACSVerse flow cytometer. Cell proliferation was analyzed using FlowJo software version 10.4.2 (FlowJo, OR, USA).

As a result, as shown in Fig. 9-1, Treg cells stimulated with anti-TIGIT agonistic antibody suppressed the proliferation of effector T cells. Stimulation of Treg cells with anti-TIGIT agonistic antibody caused activation of Treg cells.

In the same manner as in the above, Treg cells (FrI) or Treg cells (FrII) were stimulated for 6 hr with an anti-CD3 antibody and an anti-CD28 antibody in the presence of an isotype (mouse IgG1) or an anti-TIGIT agonistic antibody. Non-Treg responder CD4+ T cells (CD25-) were labeled with CTV and co-cultured with each activation-treated Treg cell fraction at a ratio of 1:1 ($1\times10^4$ each) for 96 hr. Thereafter, cell proliferation of responder T cells was analyzed using the dilution of CTV fluorescence as an index in the same manner as above. As a result, all Treg cell fractions suppressed the proliferation of responder T cells, but activation of Treg cells was enhanced more when each Treg cell fraction was activated in the presence of an anti-TIGIT agonistic antibody, and the effect was more remarkable particularly in Treg (FrI) which is also known as a naive Treg cell (Fig. 9-2).

Example 6: Effect on imiquimod-induced lupus model mice

6-8-week-old female C57BL/6JJcl mice (CLEA Japan, Inc.) were purchased and mated with mice of the same strain obtained by knocking in the human TIGIT (hTIGIT) gene by a conventional method. The resulting homozygous wild-type hTIGIT knockin mice of the same generation were used. All experiments were conducted at the Keio University School of Medicine animal facility, and were carried out in accordance with the regulations relating to animal experiments, etc. at the Keio University Faculty of Medicine.

Imiquimod-induced lupus model mice were produced by applying 5% imiquimod cream (Mochida Pharmaceutical Co., Ltd.) at 50 mg/kg to the right auricle of 10- to 12-week-old female TIGIT-KI mice three times a week. The administration experiment was performed as follows. In all cases, administration was performed twice per week. They were divided into 3 groups and analyzed on 42 days after the start of administration.

unapplied: imiquimod unapplied/intraperitoneal administration of 300 μg/ml of Isotype (mIgG1)
anti-TIGIT: imiquimod applied/intraperitoneal administration of 300 μg/ml of anti-TIGIT agonistic antibody
isotype: imiquimod applied/intraperitoneal administration of 300 μg/ml of Isotype (mIgG1)

Mouse spleen was isolated and weighed. The spleen was finely chopped, passed through a 40 um cell strainer, subjected to a hemolysis treatment using HLB solution (IBL), and the total number of splenocytes was measured. Mouse splenocytes were cultured with anti-CD16/CD32 (BD Biosciences) for 5 min at 4°C and surface staining was performed for 20 min. The medium for staining was a mixture of PBS, 0.5% BSA, and 2 mM EDTA. Each cell was defined as follows: Tern; CD44+CD62L-, Tfh; CXCR5+PD-1+, plasma cells; CD19-CD138+, GC B cells; CD95+GL7+. At the time of analysis, blood was collected and the blood anti-dsDNA antibody titer was measured using "LBIS Mouse anti-dsDNA ELISA KIT" (manufactured by Shibayagi).

As a result, as shown in Fig. 10, splenomegaly and proliferation of lymphocytes were suppressed in the anti-TIGIT agonistic antibody administration group. As shown in Fig. 11-1, administration of the anti-TIGIT agonistic antibody significantly suppressed an increase in the proportion of plasma cells in B cells. In addition, a tendency toward suppression was shown on an increase in the proportion of activated T cells in T cells, the proportions of CD4+ Effector memory T (Tem) cells and Tfh cells in CD4 positive T cells, and the proportion of germ center B cells in B cells, and an increase in the production of anti-dsDNA antibody. When the same experiment was conducted with an increased number of mice, the anti-TIGIT agonistic antibody significantly suppressed, as compared to isotype, an increase in the proportion of Tern cells and Tfh cells in CD4 positive T cells, the proportion of germ center B cells in B cells, and the proportion of plasma cells in splenocytes, and an increase in the production of anti-dsRNA antibody (Fig. 11-2).

As described above, the anti-TIGIT agonistic antibody suppressed the proliferation of lymphocytes due to a disease, particularly the proliferation of activated T cells. Furthermore, it also suppressed the activation of B cells via Tfh cells, similar to the results in the culture experiments.

Example 7: Effect on experimental autoimmune encephalomyelitis (EAE) mice

12-week-old male TIGIT-KI mice were injected subcutaneously with 200 μg of MOG$_{35-55}$ peptide (Synpeptide, Shanghai, China) and Freund's incomplete adjuvant added with 400 μg of *M. tuberculosis* (Difco Laboratories). On the same day and on day 2, 400 ng of whooping cough toxin (List Biologicals) was administered intraperitoneally. On days 0, 2, 4, 10, and 17, 100 μg of anti-TIGIT agonistic antibody or isotype (mIgG1) was administered intraperitoneally. Clinical scores were measured every day using the following method and analyzed on day 30 (0, no change; 1, tail muscle weakness; 2, tail paralysis; 3, hindlimb muscle weakness; 4, forelimb muscle weakness; 5, paralysis of forelimb; 6, dying or dead). Cells invading the brain, spinal cord, and spleen were analyzed by flow cytometry using various antibodies.

As a result, as shown in Fig. 12, the EAE clinical score was reduced in the antibody administration group. As shown in Fig. 13, the antibody-administered group tended to promote the proliferation of Treg cells in all of the brain, spinal cord, and spleen.

Results·discussion

From the above-mentioned results, the anti-TIGIT agonistic antibody demonstrated the suppressive effect on Tfh cells and the suppressive effect on B cells via Tfh cells both *in vitro* and *in vivo.* The results were confirmed that anti-TIGIT agonistic antibody actually showed a suppressive effect on effector T cells *in vitro,* and that the administration of this antibody also suggests promoted proliferation of Treg cells *in vivo.* Studies using disease model animals confirmed the efficacy of this antibody. All of the above-mentioned effects are effects that have not been seen with conventional antibodies. Suppressing activated T cells and activating Treg cells in immune/inflammatory diseases are reasonable as a therapeutic drug, and can provide a therapeutic drug for a wide range of human immune/inflammatory diseases in the future.

[Industrial Applicability]

According to the present invention, signals via human TIGIT can be activated, whereby Tfh cells can be suppressed and Treg cells can be activated. Therefore, the present invention is useful for the prophylaxis or treatment of immune/inflammatory diseases.

The present invention is based on JP 2020-171278 filed on October 9, 2020 in Japan, the contents of which are incorporated in full herein by reference.

**Claims**

1. A therapeutic agent for an immune/inflammatory disease comprising an anti-human TIGIT antibody, which activates a suppressive immune checkpoint molecule (TIGIT), as an active ingredient.

2. The therapeutic agent according to claim 1, which has Tfh cell suppressing action.

3. The therapeutic agent according to claim 1 or 2, which has Treg cell activating action.

4. The therapeutic agent according to any one of claims 1 to 3, wherein the immune/inflammatory disease is selected from the group consisting of rheumatoid arthritis, systemic lupus erythematosus, Sjogren's syndrome, systemic scleroderma, polymyositis, dermatomyositis, IgG4-associated disease, Takayasu arteritis, giant cell arteritis, polyarteritis nodosa, ANCA-associated vasculitis, mixed connective tissue disease, spondylarthritis, Behcet's disease, adult Still's disease, multiple sclerosis, optic nerve myelitis, myasthenia gravis, primary biliary cirrhosis, non-alcoholic fatty liver diseases, primary sclerosing cholangitis, autoimmune hepatitis, ulcerative colitis, Crohn's disease, psoriasis (psoriatic arthritis), vitiligo vulgaris, bullous pemphigoid, circular shape alopecia, idiopathic dilated cardiomyopathy, type 1 diabetes, Graves' disease, chronic thyroiditis, IgA nephropathy, membranous nephropathy, hemolytic anemia, and idiopathic thrombocytopenic purpura.

5. The therapeutic agent according to any one of claims 1 to 4, wherein the anti-human TIGIT antibody is an antibody binding to human TIGIT (SEQ ID NO: 1).

6. The therapeutic agent according to claim 5, wherein in the antibody,

   a) the third CDR of the heavy chain variable region comprises the amino acid sequence of SEQ ID NO: 4, and
   b) the third CDR of the light chain variable region comprises the amino acid sequence of SEQ ID NO: 6.

7. The therapeutic agent according to claim 5, wherein the antibody comprises

   a) a heavy chain variable region comprising the following:

   i) the first CDR comprising the amino acid sequence of SEQ ID NO: 2;
   ii) the second CDR comprising the amino acid sequence of SEQ ID NO: 3; and

b) a light chain variable region comprising the following:

  i) the first CDR comprising the amino acid sequence of SEQ ID NO: 5;
  ii) the second CDR comprising the amino acid sequence of tyrosine-alanine-serine.

8. The therapeutic agent according to claim 5, wherein the antibody further comprises Fc domain.

9. The therapeutic agent according to claim 8, wherein the Fc domain is derived from human.

10. The therapeutic agent according to claim 8 or 9, wherein the Fc domain is a mutated Fc domain.

11. An antibody:

  a) whose third CDR of the heavy chain variable region comprises the amino acid sequence of SEQ ID NO: 4, and
  b) whose third CDR of the light chain variable region comprises the amino acid sequence of SEQ ID NO: 6.

12. The antibody according to claim 11, which binds to human TIGIT (SEQ ID NO: 1).

13. The antibody according to claim 11, comprising

  a) a heavy chain variable region comprising the following:

    i) the first CDR comprising the amino acid sequence of SEQ ID NO: 2;
    ii) the second CDR comprising the amino acid sequence of SEQ ID NO: 3; and

  b) a light chain variable region comprising the following:

    i) the first CDR comprising the amino acid sequence of SEQ ID NO: 5;
    ii) the second CDR comprising the amino acid sequence of tyrosine-alanine-serine.

14. The antibody according to claim 11, which further comprises Fc domain.

15. The antibody according to claim 14, wherein the Fc domain is derived from human.

16. The antibody according to claim 14, wherein the Fc domain is a mutated Fc domain.

17. An isolated nucleic acid encoding the antibody according to claim 11.

18. A host cell comprising the isolated nucleic acid according to claim 17.

19. A method of producing the antibody according to claim 11, comprising a step for culturing the host cell according to claim 18 under conditions that allows the production of the antibody.

[Fig. 1]

CD2F1

[Fig. 2]

## M1-8

FL4 Log.: FL4 Log

——— Doxycyclin+/hTIGIT_CHO cell
········ Doxycyclin-/hTIGIT_CHO cell

[Fig. 3]

MSBA43 (P.C.)      M1-8

FL4 Log.: FL4 Log     FL4 Log.: FL4 Log

——— 500 ng/mL anti-hTIGIT mAb
——— 50 ng/mL anti-hTIGIT mAb
——— 5 ng/mL anti-hTIGIT mAb
——— 0 ng/mL anti-hTIGIT mAb
········ No hTIGIT-hFc

Anti-hTIGIT antibody
(positive control)

[Fig. 4]

M1-8

agonistic activity
antagonistic activity
cytotoxicity/growth inhibition activity

[Fig. 5]

[Fig. 6]

[Fig. 7-1]

[Fig. 7-2]

[Fig. 8]

[Fig. 9-1]

[Fig. 9-2]

[Fig. 10]

[Fig. 11-1]

[Fig. 11-2]

[Fig. 12]

[Fig. 13]

## INTERNATIONAL SEARCH REPORT

| International application No. |
|---|
| **PCT/JP2021/037471** |

**A.    CLASSIFICATION OF SUBJECT MATTER**

*A61K 39/395*(2006.01)i; *A61P 29/00*(2006.01)i; *A61P 37/02*(2006.01)i; *C07K 16/28*(2006.01)i; *C12N 15/13*(2006.01)i
FI:    A61K39/395 N; A61K39/395 D; A61P37/02; A61P29/00; C07K16/28; C12N15/13 ZNA

According to International Patent Classification (IPC) or to both national classification and IPC

**B.    FIELDS SEARCHED**

Minimum documentation searched (classification system followed by classification symbols)

A61K39/395; A61P29/00; A61P37/02; C07K16/28; C12N15/13

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); UniProt/GeneSeq

**C.    DOCUMENTS CONSIDERED TO BE RELEVANT**

| Category* | Citation of document, with indication, where appropriate, of the relevant passages | Relevant to claim No. |
|---|---|---|
| X | WO 2015/143343 A2 (THE BRIGHAM AND WOMEN'S HOSPITAL, INC.) 24 September 2015 (2015-09-24) | 1-5, 8 |
|   | paragraphs [0226]-[0229], example 7, claims 94-106 |   |
| Y |   | 9, 10 |
| A |   | 6, 7, 11-19 |
| Y | JP 2006-512407 A (XENCOR INC) 13 April 2006 (2006-04-13) | 9, 10 |
|   | examples 1-12 |   |
| A | JP 2008-544746 A (ZYMOGENETICS, INC) 11 December 2008 (2008-12-11) | 1-19 |
|   | paragraphs [0315]-[0329] |   |
| A | JP 2011-523034 A (GENENTECH, INC) 04 August 2011 (2011-08-04) | 1-19 |
|   | claims 1-38, paragraph [0051] |   |

☐ Further documents are listed in the continuation of Box C.        ☑ See patent family annex.

| | |
|---|---|
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **26 November 2021** | **07 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2021/037471**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**EP 4 226 943 A1**

**INTERNATIONAL SEARCH REPORT**
Information on patent family members

International application No.

**PCT/JP2021/037471**

| Patent document cited in search report | | | Publication date (day/month/year) | Patent family member(s) | | | Publication date (day/month/year) |
|---|---|---|---|---|---|---|---|
| WO | 2015/143343 | A2 | 24 September 2015 | US 2017/0107300 A1 paragraphs [0245]-[0248], example 7, claims 94-106 EP 3119913 A1 | | | |
| JP | 2006-512407 | A | 13 April 2006 | US 2004/0132101 A1 examples 1-12 WO 2004/029207 A2 EP 1553975 A2 CN 1705491 A KR 10-2005-0071523 A | | | |
| JP | 2008-544746 | A | 11 December 2008 | US 2007/0054360 A1 paragraphs [0312]-[0326] WO 2006/124667 A2 EP 1891107 A2 | | | |
| JP | 2011-523034 | A | 04 August 2011 | US 2009/0258013 A1 paragraph [0093], claims 1-38 WO 2009/126688 A2 EP 2279412 A2 KR 10-2010-0135892 A CN 104655854 A | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006124667 A
- WO 2009126688 A
- US 841654
- US 2004013210 A
- US 20050054832 A
- US 20060024298 A
- US 20060121032 A
- US 20060235208 A
- US 20070148170 A
- US 4937190 A
- JP 2020171278 A

**Non-patent literature cited in the description**

- *PLoS ONE,* 2008, vol. 3, e2532
- *Stem Cells,* 2007, vol. 25, 1707
- *Science,* 1952, vol. 122, 501
- *Virology,* 1959, vol. 8, 396
- *The Journal of the American Medical Association,* 1967, vol. 199, 519
- *Proceeding of the Society for the Biological Medicine,* 1950, vol. 73, 1
- *Methods in ENZYMOLOGY,* vol. 70
- *Immunochemical Techniques,* vol. 73
- *Immunochemical Techniques,* vol. 74
- *Immunochemical Techniques,* vol. 84
- Selected Immunoassays. *Immunochemical Techniques,* vol. 92
- Monoclonal Antibodies and General Immunoassay Methods. *Immunochemical Techniques,* vol. 121
- Hybridoma Technology and Monoclonal Antibodies. Immunochemical Techniques. Academic Press
- Remington's Pharmaceutical Science. 1980